# EUROPEAN PATENT SPECIFICATION

(11) **EP 1 397 513 B1**
(45) Date of publication and mention of the grant of the patent: **25.05.2005**
(21) Application number: 02742552.9
(22) Date of filing: 21.06.2002
(51) Int. Cl.: C12Q 1/68

(54) **NOVEL TECHNOLOGY FOR GENETIC MAPPING**
NEUARTIGE TECHNOLOGIE ZUR GENETISCHEN KARTIERUNG
NOUVELLE TECHNOLOGIE DE CARTOGRAPHIE GENETIQUE

(30) Priority: 21.06.2001 GB 0115194
(43) Date of publication of application: 17.03.2004
(73) Proprietor: K.U. Leuven Research & Development, 3000 Leuven (BE)
(72) Inventor: THEVELEIN, Johan, B-3052 Blanden (BE); VAN DIJCK, Patrick, B-3271 Zichem (BE); MA, Pingsheng, School of Chem. Engin.&Technology, Weijin Lu 92 300072 Tianjin (CN); DUMORTIER, Françoise, B-3052 Blanden (BE); BROOTHAERTS, Wim, B-2400 Mol (BE)
(74) Representative: Bird, Ariane
(86) International application number: PCT/BE2002/000106
(87) International publication number: WO 2003/002709

(56) References cited:
- SHOEMAKER D D ET AL: "QUANTITATIVE PHENOTYPIC ANALYSIS OF YEAST DELETION MUTANTS USING A HIGHLY PARALLEL MOLECULAR BAR-CODING STRATEGY" NATURE GENETICS, NEW YORK, NY, US, vol. 14, December 1996 (1996-12), pages 450-456, XP002043431 ISSN: 1061-4036
- HENSEL M ET AL: "WHOLE GENOME SCAN FOR HABITAT-SPECIFIC GENES BY SIGNATURE-TAGGED MUTAGENESIS" ELECTROPHORESIS, WEINHEIM, DE, vol. 19, no. 4, April 1998 (1998-04), pages 608-612, XP001085303 ISSN: 0173-0835
- LASHKARI ET AL: "WHOLE GENOME ANALYSIS: EXPERIMENTAL ACCESS TO ALL GENOME SEQUENCED SEQGMENTS THROUGH LARGER-SCALE EFFICIENT OLIGONUCLEOTIDE SYNTHESIS AND PCR" PROCEEDINGS OF THE NATIONAL ACADEMY OF SCIENCES OF USA, NATIONAL ACADEMY OF SCIENCE. WASHINGTON, US, vol. 94, August 1997 (1997-08), pages 8945-8947, XP002100295 ISSN: 0027-8424

## Description

### Field of the invention

The invention describes a novel method for the mapping of genes in sexually reproducing eukaryotic organisms. The invention is distinct from other mapping methods by the use of crossing a strain with a phenotype of interest with a modified strain that contains a set of uniformly distributed unique marker sequences in silent regions of the genome. The invention further describes the generation of these engineered strains, the use of these strains in gene mapping and methods and materials to detect the unique marker sequences.

### Background of the invention

Genetic mapping is commonly used in sexually reproducing eukaryotic organisms as a means to identify genes that are responsible for phenotypic traits (Dear P.H., Ed., 1997, Genome mapping, A practical approach, IRL Press, Oxford). There exist a large number of genetic mapping technologies. In essence all technologies aim to locate the genetic determinants) for a specific phenotypic property somewhere in the genome based on linkage analysis with a marker of which the location is known. Linkage with markers of which the precise location is not known but which can be traced much more easily than the phenotypic property itself is often used in breeding experiments. Genetic mapping is widely used in medical biology to identify genes responsible for human diseases (Ott J., 1991, Analysis of human genetic linkage. John Hopkins University Press, Baltimore). In agricultural research it is used for identifying genes in domesticated animals and crop plants which are responsible for a variety of properties that are directly or indirectly important for productivity or performance (Paterson A.H., Ed., 1998, Molecular dissection of complex traits, CRC Press, Boca Raton). It is used with the same purpose in eukaryotic micro-organisms like the yeast *Saccharomyces cerevisiae* (Spencer et al. 1983, Yeast genetics, fundamental and applied aspects, Springer Verlag, New York). Genetic mapping is intensively used in biological research, in particular in model organisms *like Saccharomyces cerevisiae* (Johnston J.R., 1994, Molecular genetics of yeast, a practical approach, IRL Press, Oxford), *Schizosaccharomyces pombe* (Cox B.S. 1995, In The Yeasts, Vol.6 Yeast genetics, Academic Press, San Diego), *Arabidopsis thaliana (thale cress)* (Wilson Z.A., 2000, *Arabidopsis* a practical approach, Oxford University Press), *Drosophila melanogaster* (Greenspan R.J., 1997, Fly pushing: the theory and practice of *Drosophila* genetics, Cold Spring Harbor Laboratory Press), the nematode *Caenorhabditis elegans* (Riddle D.L. et al., Eds., 1997, *C. elegans* II, Cold Spring Harbor Laboratory Press, Cold Spring Harbor), *zebrafish (Brachydanio rerio)*(Detrich H.W. III, Westerfield M., and Zon L.I., Eds, The zebrafish: genetics and genomics, Academic Press, San Diego) *and mouse (Mus musculus*)(Lyon M.F. et al., Eds. 1996, Genetic variants and strains of the laboratory mouse, 3d Ed., Oxford University Press, New York) to correlate phenotypes with the position in the genome of genes responsible for the phenotype. Model organisms have been chosen to a large extent based on the ease with which genetic experiments, including genetic mapping, can be performed.

In genetic mapping a variety of genetic markers is used. A crucial characteristic of a genetic marker is the ease with which it can be scored. The more markers can be scored with the least experimental effort the better. All existing genetic mapping technologies make use of either mutations conferring specific phenotypic properties or of natural DNA sequence variation. Easily scoreable mutations such as auxotrophic and resistance mutations are highly preferred because they require least experimental effort for their detection (Mortimer R.K. and Schild D. 1981, In: The molecular biology of the yeast *Saccharomyces.,* Life cycle and inheritance, Strathern J.N., Jones E.W. and Broach J.R., Eds., Cold Spring Harbor Laboratory, New York, pp.11-26). An important disadvantage of auxotrophic, resistance and other mutations causing a specific phenotype is that the mutant phenotype caused by the marker mutation might interfere with the phenotype of interest. For instance, auxotrophic mutations in yeast interfere with the growth rate even if the medium is supplemented with the nutrient for which the strain is auxotrophic. Since many marker mutations are required for genetic mapping, the accumulation of multiple mutations affecting the phenotype in a single strain easily generates unexpected side-effects on many other properties of the strains, in particular properties of commercial importance such as growth and yield. Hence, for that reason the use of marker mutations that influence the phenotype is undesirable in genetic mapping. Methods employing natural DNA sequence variation include detection of restriction fragment length polymorphisms (RFLP's), Random amplification of polymorphic DNA (RAPD), amplified fragment length polymorphisms (AFLP's), single nucleotide polymorphisms (SNP's), microsatellite repeat sequences, fluorescent in situ hybridization (FISH) for mapping of clones to chromosomes (Burow M.D. and Blake T.K. 1998, In: Molecular dissection of complex traits, Paterson A.H. Ed., CRC Press, Boca Raton, pp.13-29; Montagutelli X. In: Systematic approach to evaluation of mouse mutations, Sundberg J.P. and Boggess D., Eds., pp. 15-33; Karp A. and Edwards K.J., In: Caetano-Anollés G. and Gresshoff P.M., Eds., 1997, DNA Markers, Protocols, applications and overviews, Wiley-VCH, New York, pp.1-13). Up to now artificial DNA sequences, combinations of artificial DNA sequences and part of the adjacent DNA sequence, or combinations of artificially introduced natural sequences and part of the adjacent DNA sequence, have never been used as genetic markers. All genetic mapping makes use of DNA sequence variation present in natural isolates or cultivated strains of organisms. At most it makes use of mutations that have been introduced because they confer a specific, usually easily traceable phenotype.

A major development in genetic mapping is the use of natural molecular variation in the DNA sequence between organisms as a source of genetic markers (Birren et al. 1999, Genome analysis, a laboratory manual. Vol.4. Mapping genomes. Cold Spring Harbor Laboratory Press, Cold Spring Harbor). DNA polymorphisms have the advantage that they can much more easily be scored than phenotypic properties. This is particularly important for the mapping of polygenic traits, because the workload involved is much larger and because the contribution to the determination of the phenotype is split over different genes, giving each gene a fractional effect on the phenotype which is more easily influenced or overshadowed by phenotypic side-effects caused by phenotypic marker mutations. As for molecular markers, single nucleotide polymorphisms (SNP's) are an important example because of their widespread occurrence. In practice, when two unrelated strains are crossed, SNP's can provide thousands of genetic markers. Recently, allelic variation between two strains of unrelated origin has been used in yeast to provide genetic markers on a genome-wide scale for mapping of phenotypic properties. In this case SNP's provided 3714 usable genetic markers (Winzeler E.A. et al. 1998, Science 281, 1194-1197). In spite of this high density, the largest gap between two markers was still 59 kb. Obviously, in more closely related strains the gaps would be much larger. This 'allelic variation linkage mapping' method was made possible because of the availability of the complete yeast genome sequence and of micro-arrays with oligonucleotides complimentary to short parts of all yeast genes, which were originally constructed for gene expression purposes. The availability of thousands of naturally occurring SNP's for mapping purposes has attracted most attention and efforts of the genetics research community to 'SNP-mapping'. Moreover, the introduction of artificial DNA sequences, especially in precisely predetermined positions in the genome is difficult with most eukaryotic organisms. Only in yeast this is relatively easy. However, because of the large number of markers required to cover the genome completely, the introduction of all markers in a single strain is still a huge work. Even if the markers are introduced in a parallel fashion in different strains simultaneously, the strains still have to be crossed with each other to accumulate all markers into a single strain. Because the markers are always segregating out in crosses between parental strains, this is also a very labour-intensive task. Moreover, it requires an organism with a convenient sexual reproduction cycle and a short generation time. These are probably the reasons why nobody before has considered the use of artificial markers for genetic mapping purposes and in particular why nobody has considered covering the genome completely with a large number of genetic markers. Up to now, all genetic mapping has been carried out with naturally occurring sequence variation or with mutations conferring a specific phenotype. In the latter case, easily screenable phenotypes such as auxotrophic, antibiotic resistance or temperature sensitive mutations have been used. Collections of strains with a number of such mutations that cover the genome to a certain extent have been made and used for mapping with low resolution. For instance, in the yeast *Saccharomyces cerevisiae,* a collection of nine strains, which in total have 66 markers spaced approximately 50 cM apart over the entire genome is available (Mortimer R.K. and Schild D. 1981, In: The molecular biology of the yeast *Saccharomyces.,* Life cycle and inheritance, Strathern J.N., Jones E.W. and Broach J.R., Eds., Cold Spring Harbor Laboratory, New York, pp.11-26). The use of such strains for mapping is very labour intensive since crosses have to be made with nine different strains and since all the mutations have to be scored using the specific phenotype that they cause. The strains are also not isogenic.

A major disadvantage of all mapping methods based on natural genetic variation is that they require the use of unrelated or non-isogenic strains, since DNA sequence differences are required for the construction of the genetic map. As a result, in all such methods the genetic map is not independent from the phenotype of the organism. This problem can be illustrated with an example in *C. elegans* where a special isolate from a Hawaiian island was used for mapping because it showed a uniformly high density of DNA polymorphisms (Wicks S.R. et al. 2001, Nature Genetics 28, 160-164). About 6200 DNA polymorphisms were identified, of which 4670 were single-base pair substitutions and 1552 small deletions and insertions. However, there were also more than 400 insertions and deletions of two and more basepairs. It is clear that such a large amount of DNA sequence variation will affect many phenotypes. It was also noted in this report that effects of the genetic background of the Hawaiian strain had already been observed for some important phenotypes. To solve this problem it was suggested to develop a collection of inbred hybrid strains with a large contiguous tract of the DNA from the Hawaiian strain in the region of the target mutation. It is clear that this involves a large additional workload for the mapping of a mutation and that it also does not guarantee the complete elimination of background effects on the phenotype of interest. This problem is especially important for genetic analysis of multigenic phenotypes where different mutations contribute to the establishment of the phenotype and where background effects arising from the SNP's and larger DNA polymorphisms can easily make proper genetic analysis impossible. The presence of thousands of SNP's in the background also complicates identification of the mutation(s) responsible for the phenotype. It is to be emphasised that genetic mapping is not a final goal anymore in scientific research, it is only an intermediate step towards identification of the gene(s) involved in a phenotype of interest. When the background of the strain contains thousands of mutations that are irrelevant for the determination of the phenotype, this complicates the final identification of the relevant mutation(s). This is most problematic for multigenic properties and in particular for multigenic properties where the mutations causing the phenotype are interdependent. Genetic analysis of multigenic properties is very cumbersome with all existing genetic mapping methods.

Different natural isolates of organisms, especially from unusual habitats, often display special phenotypic properties. When the mutations or novel genes involved in these properties are to be mapped and identified, the strain has to be crossed with an unrelated strain differing in a sufficient number of molecular markers. These markers are used to construct the genetic map. For each novel strain isolated the molecular markers have to be checked again to determine whether they differ in the new strain and the mapping strain. As a result, for each novel strain investigated the genetic map has to be established again.

Artificial DNA sequences have never been used as genetic markers in mapping technologies. However, they have been used as tags in some other applications. We provide a number of examples. A well-known application is tagged mutagenesis, for which a broad range of transposable elements, including specifically engineered transposons, is used, (Garfinkel D.J. et al. 1998, In: Methods in Microbiology Vol.26, Yeast gene analysis, Brown A.J.P. and Tuite M.F., Eds., Academic Press, San Diego; Walbot V. 1992, Ann. Rev. Plant Physiol. Plant Mol. Biol. 43, 49-82). In this case a strain is transformed with the transposable element with the aim of introducing it preferentially into an open reading frame or at least into part of a DNA sequence (promoter, terminator) that affects the expression of an open reading frame. The transposon serves as a tag to identify the position of the mutation in the genome by sequence analysis of the DNA adjacent to the transposon. Alternatively, T-DNA tags may be introduced in the genome using one of several genetic transformation methodologies utilizing the bacterium *Agrobacterium*. A second example concerns the use of unique, short oligonucleotides, called 'signatures', which are inserted randomly into a strain. The resulting 'signature-tagged mutants' are subjected to a selection procedure after which the signature tag can be used to rapidly identify the insertion position of the tag in the genome by sequence analysis of the adjacent DNA (Hensel M., 1998, Electrophoresis 19, 608-612). A third example concerns the use of a comprehensive collection of deletion mutants of the yeast *Saccharomyces cerevisiae* in which a different open reading frame is completely deleted in each strain. Each strain contains a specific tag adjacent to the deleted gene. In experiments where the whole collection of deletion mutants is grown under selective conditions in order to enrich for mutants affected in a certain phenotype, the tags are afterwards used to identify the gene that has been deleted in the selected strain(s) by sequence analysis (Shoemaker DD, Lashkari DA, Morris D, Mittmann M and Davis RW, 1996, Nature Genetics 14, 450-456).

To facilitate the identification of molecular markers linked to a certain phenotype, DNA pooling of segregants displaying different phenotypes or extremes of the same phenotype (in the case of quantitative trait loci) has been used. This method has been called 'bulked segregant analysis' (BSA). It strongly reduces the workload involved in the determination of large numbers of molecular markers. Up to now this approach has been used mainly to identify molecular markers linked to a certain phenotype, gene or genomic region of interest (Giovannoni J.J. et al. 1991, Nucleic Acids Res. 19, 6553-6558; Michelmore R.W. et al. 1991, Proc. Natl. Acad. Sci. USA 88, 9828-9832). It has also been used to map mutations in specific areas of the genome using multiple rounds of detection of molecular markers (Korswagen H.C. et al. 1996, Proc. Natl. Acad. Sci. USA 93, 14680-14685; Wicks S.R. et al. 2001, Nature Genetics 28, 160-164). Bulked segregant analysis has never been performed with artificial markers and never with a methodology allowing simultaneous detection of all markers covering the whole genome with high resolution.

For identification of mutant genes in micro-organisms, such as yeast, complementation with a genomic library is often used (Johnston J.R.1988. In: Yeast, A practical approach, Campbell I. and Duffus J.H., Eds., IRL Press, Oxford, p.107-123.) For this purpose the genomic DNA of an organism is first fragmented with a restriction enzyme. Subsequently, the fragments are inserted into a vector (usually after selection for a certain size range) and the resulting plasmids transformed into a recipient organism, usually *Escherichia coli* where each cell contains only one type of plasmid. The library is propagated as a mixture of recipient cells, e.g. *E. coli* transformants. This procedure has several disadvantages. First, there is never a guarantee for completeness of the library when it is constructed. To make the gene library as complete as possible many more transformants have to be obtained during the preparation of the library than theoretically needed to cover the genome. Second, propagation of the library inevitably results in degeneration of the library, because plasmids get lost randomly. Third, when the library is used, large numbers of transformants have to be generated in order to approach statistically complete coverage of the genome in the transformants. Fourth, even if large numbers of transformants are obtained, whether the genome has been covered completely is never sure and can not be assessed.

### Summary of the invention

The present invention provides a method of genetic mapping that uses markers that are entirely independent of the phenotype of the organism. It allows isogenic strains to be used for isolation of mutants and subsequent mapping. The markers should be evenly spaced over the whole genome and the number should be high enough to detect linkage with any gene and not higher than necessary to avoid collection of useless information. The markers should be detectable simultaneously with a simple experiment. In one aspect of the present invention man made or artificial oligonucleotides as markers allow high-resolution genetic mapping with one single cross and rapid scoring of markers covering the whole genome.

The artificial markers are preferably absent in new natural isolates of the organism. The present invention also includes within its scope cases where one or more artificial markers by coincidence are identical with a DNA sequence in a novel strain. This can be checked with the DNA of the novel strain and when the density of the artificial markers is high, loss of one or even a few markers will not prevent high-resolution mapping.

The invention relates to modified non-human eukaryotic organisms. Said organisms are engineered by man-made intervention, by which the DNA sequence of the organism is changed by a plurality of one or more modifications. These modifications are preferably substantially equally distributed throughout a part or several parts or the entire length of one or more or all of the chromosomes of the genome.

The invention relates to modified organisms where the modifications are preferably introduced into silent regions of the genome, for example, outside of the coding regions and the regulatory parts of the genes. More preferably the modifications occurs outside more than 80 % of the coding an/or regulatory regions. Even more preferably the modifications occurs outside more than 90 % of the coding and/or regulatory regions. Even more preferably the modifications occurs outside more than 95 % of the coding and/or regulatory regions. Most preferably the modifications occurs outside more than 99 % of the coding and/or regulatory regions.

The use of isogenic strains which only differ in artificially introduced markers, as described in one aspect of the present invention, entirely overcomes the problem of using heterogenic strains as discussed above. It allows to identify the mutation(s) responsible for the phenotype by simple sequence analysis of the area to which the mutation has been mapped. The use of isogenic strains is strongly preferred in genetic mapping because it also avoids effects of background mutations (such as SNP's) on the phenotype of interest. Especially for polygenic traits, genetic background effects can make genetic analysis very difficult and often impossible in practice. Isogenic strains (also called completely homozygous or pure lines) are strains that have in principle the same DNA sequence in the whole genome. Hence, the presence of markers in isogenic strains is actually a contradiction. Markers are always based on DNA sequence variation. As explained in this invention the closest possibility to the ideal situation of genetic mapping with isogenic strains, is the usage of artificial DNA sequences inserted in neutral positions in the genome so that they have no effect on the phenotype. In accordance with the present invention the term "isogenic" is used in this wider sense, i.e. to include genomic sequences which differ in silent regions but have the same active gene sequences.

The invention relates to modified organisms where the modifications can occur with a ratio of at least 1 modification per 100 genes.

For this invention is preferable that the modifications result in an organism that is still viable and able to reproduce sexually.

The invention relates to modified organisms where the modifications are preferably substantially equally distributed throughout the genome and preferably do not modify the phenotype with respect to the unmodified organism.

The invention relates to organisms wherein the modifications occur preferably at site specific places or wherein the modifications occur in a site directed manner.

It will be appreciated that a limited number of local deviations in the regular spacing of markers will be tolerated and that a limited number of modifications that occur within genes or cause a slight change in phenotype that is not relevant to the phenotype to be mapped is acceptable. It will also be appreciated that organisms where modifications for instance only cover a limited number of chromosomes or where the distributed modifications occur in discrete regions of a chromosome also fall within the scope of the invention.

The invention relates further to an engineered organism where the modifications are insertions, deletions or substitutions and where these modifications can occur in combination with one or more additional modifications such as a restriction enzyme recognition site, a nucleotide tag, sequences flanked with inverted repeats such as transposons, sequences flanked with long terminal repeats of a retrovirus. The modifications can also be mobile genetic elements such as transposons, or the footprints that remain after the excision of a mobile genetic element.

The engineered organism of this invention can be obtained by a method such as homologous recombination, viral infection, random integration or processes related to *Agrobacterium*-mediated transformation.

The engineered organism of this invention can also be obtained by interfering with the process of mobilisation of mobile genetic elements by using a strain with an elevated or reduced level of mobilisation, or by crossing strains of a species that would not be able to mate under natural conditions.

The engineered organism of this invention can be a member of the taxonomic groups of fungi including filamentous fungi, non vascular and vascular plants, invertebrates, arthropods, nematodes, vertebrates or mammals; preferably it is one of the model organisms that are being used to study these groups such as *Saccharomyces cerevisiae, Schizosaccharomyces pombe, Aspergillus nidulans, Neurospora sp., Caenorhabditis elegans, Physcomitrella sp., Arabidopsis thaliana, Oryza sativa, Drosophila melanogaster, Brachydanio rerio* or *Mus musculus*.

The present invention relates in the first instance to the yeast *Saccharomyces cerevisiae*, but the methodology can be applied to other viable eukaryotic organisms that are able to reproduce sexually.

The engineered organism of this invention has preferably a genome that is completely sequenced or of which a substantial part is sequenced or that is expected to be sequenced in the near future.

The modifications engineered in the genome of the organism of this invention lead to the presence of artificial marker sequences in the genome. These marker sequences either being generated only by insertion of the sequences themselves or by bridging the sequence introduced and a sequence located closely in the adjacent genomic DNA.

The invention relates to a method for mapping genes by crossing an organism with a phenotype of interest with the modified organism of this invention, selecting segregants of the progeny with the phenotype of interest. DNA is isolated from the selected segregants and after an optional pooling step of the isolated DNA the presence of one or more markers in the DNA of the selected segregants which are close to genes determining the phenotype are detected. As will be understood from the concept of recombination between chromosomes during sexual reproduction, the chance that a given marker sequence in the close proximity of a gene that causes a phenotype of interest will be transferred from the marker strain to the segregant organism with the phenotype of interest by recombination is decreasing the closer the distance is between the marker sequence and the gene of interest. Therefore the new mapping technology with the aid of the artificially introduced marker sequences is called AMTEM™ (Artificial Marker Track Exclusion Mapping).

The invention also relates to a collection of engineered strains bearing artificial markers that individually cover only part of the genome but together cover the whole genome of the species. The invention relates to a method where the markers are detected by the hybridisation of the DNA of segregants with the phenotype of interest to oligonucleotides attached to a matrix such as in a micro-array.

The oligonucleotides mentioned can eventually be longer than the oligonucleotides used for the modifications, but can also be shorter. This difference in length can have its importance, for instance, for an optimal hybridisation.

Alternatively the detection of the markers can be performed with a set of primers to be used in a polymerase chain reaction, including so-called real-time polymerase chain reaction and preferably in multiplex reactions encompassing primer pairs for several markers simultaneously.

The modified organism enables the mapping of a gene with a single cross. Phenotypes can be mapped that are caused by a mutation in a single gene or by mutations in several genes that are required simultaneously to observe the phenotype. Furthermore the modified organism can be used to map phenotypes that are caused by the deletion of regions of the genome. Further the modified organism can be used to map phenotypes that are caused by a number of mutations each mutation being able to produce the phenotype separately.

The present invention, applied to yeast, has applications in industries such as breweries, bakeries, wineries, and in the production of other alcoholic beverages and in the production of alcohol, but also in industries which use yeast as a tool for expression of heterologous proteins, for the production or modification of small molecules and in industries which use yeast for the screening of drugs or other pharmaceutical applications.

The invention also relates to strains with marker sequences where the strains will be maximally mutagenised. This results in the generation of numerous different phenotypes. The mapping technology of this invention allows identification of the genomic position of all mutated genes that are involved in the generation of a particular phenotype of interest.

The invention also relates to the use of the DNA or fragments of the DNA from the modified organism. One particular aspect of the invention relates to a modified organism where the modification is flanked by a rare restriction site. This enables the construction of a genomic library, each clone of this library having one of the introduced markers and the genomic DNA in between two adjacent markers.

The invention further relates to the use of the mapping methods of the present invention to isolate and purify genes which are identified using the method of the present invention, to generate vectors comprising said isolated and purified genes, to generate host cells to which such vectors are introduced, to the production.
The invention further relates to the use of the mapping methods in order to generate an eukaryotic organism wherein a mapped gene has been introduced.

### Brief description of the figures

Figure 1 shows an overview of Artificial Marker Track Exclusion Mapping (AMTEM™) technology, representing (A) the occurrence of markers in a chromosome, (B) the distribution of markers over the yeast genome, and (C) a presentation of an array with markers according to an embodiment of the present invention.
Figure 2 shows the mapping of a single mutation in an accordance with an embodiment of the present invention.
Figure 3 presents the mapping of two mutations in accordance with an embodiment of the present invention.
Figure 4 shows a mapping of a phenotype occurring in a multiply mutated strain according to an embodiment of the present invention.
Figure 5 shows the mapping of a single mutation in accordance with an embodiment of the present invention , wherein an artificially marked strain is mutagenised in order to obtain a phenotype which subsequently is mapped.
Figure 6 shows the introduction of rare restriction sites into an artificial marker and the subsequent generation of a genomic library in accordance with an embodiment of the present invention.
Figure 7 shows a strategy for introducing an artificial marker into the yeast genome, in accordance with an embodiment of the present invention.
Figure 8 shows detection of inserted markers in accordance with an embodiment of the present invention.
Figure 9 shows (A) an overview of different strains comprising clusters of markers (strains indicated by vertical lanes), B and C represent alternative approaches to accumulate markers in accordance with an embodiment of the present invention.
Figure 10 shows an embodiment of the present invention of a crossing strategy (panel A) to introgress the markers of partially marked strains into a single strain containing all parental markers. Panel B shows the occurrence of markers in different segregants of the final cross. Panel C shows growth rate analysis of a marked strain compared to a wild type strain.
Figure 11 shows an embodiment of the present invention, Panel A shows a PCR amplification strategy for the detection of an introduced marker. Panel B shows the detection of markers by multiplex PCR reactions on strain STW110.
Figure 12 shows exclusion of artificial markers located adjacent to one (B,C) or two mutations (D) in accordance with an embodiment of the present invention.
Figure 13 shows an example of a multiply-mutated yeast strain containing twelve gene deletions in accordance with an embodiment of the present invention. The presence of the mutations (deletions) is analysed by multiplex PCR, employing primer pairs for six ORF mutations in each reaction.

### Description of the Invention

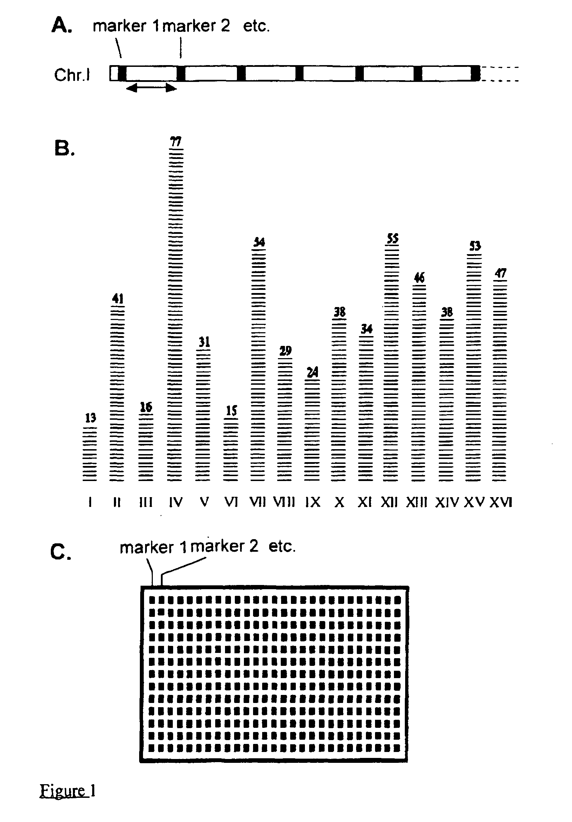

The present invention will be described with reference to certain embodiments and to certain drawings but the present invention is not limited thereto but only by the claims. In particular reference will be made to introducing so-called artificial sequences but the present invention is not limited thereto. For example it includes any suitable form of modification such as deletions and includes the conscious use of existing modifications in natural strains by cross-breeding these into a single strain.
One aspect of the present invention is concerned with a genetic mapping technology which makes use of artificial DNA sequences introduced into the genome of an organism to make an artificially marked strain. Moreover, a large number of such artificial sequences are preferably introduced into the genome of one strain so that the whole genome is in principle covered completely with artificial genetic markers. With completely is meant that if a mutation would be introduced at a random position into the genome of this artificially marked strain, it would in principle be tightly linked genetically to at least one marker, whatever its position in the genome. Hence, the whole genome of the artificially marked strain is covered with a 'track of artificial markers'. Preferably there is within the genome, a chromosome or a part thereof being modified, on average, preferable one modification for about every 100 genes, more preferable one modification for about every 10 genes, and even more preferable one modification for less than 10 genes. Subsequently, the artificially marked strain is crossed with a strain differing in a specific phenotypic trait of interest. Segregants of the cross are isolated, scored for the phenotype of interest and the DNA of all the segregants displaying the property of interest is pooled. The pooled DNA is subsequently used for the detection of the markers using any one of several methods available. Since the genes responsible for the phenotypic trait of interest can only be derived from the unmarked strain, the markers closest to these genes will be absent. Hence, in the track of artificial markers covering the genome gaps will be present where the markers closest to each gene of interest will be excluded. Therefore we have called the new technology 'Artificial Marker Track Exclusion Mapping' or 'AMTEM™'. The principle of the AMTEM™ technology is explained with an example in the Figures 1 to 4.
Figure 1 shows an overview of the Artificial Marker Track Exclusion Mapping (AMTEM™) technology. For application of the AMTEM™ technology a specially marked strain is constructed with artificial DNA sequences (markers) introduced into the genome (A). For instance, a yeast strain is constructed in which specific 20-mer oligonucleotides are introduced at a distance of about 20 kb from each other, which in yeast equals an interval containing about 10 genes. The specific markers are introduced into all yeast chromosomes (I to XVI), which means that about 600 markers have to be introduced since the genome has a length of about 12 Mb and contains about 6000 genes (B; each horizontal line denotes a marker and the total number of markers in each chromosome is shown with a number). The presence of the markers can be detected by several techniques of which a micro-array containing the compliments of the markers and to which the genomic DNA is hybridised is the most convenient (C). Each black field on the array signals the presence of the corresponding marker in the genomic DNA.

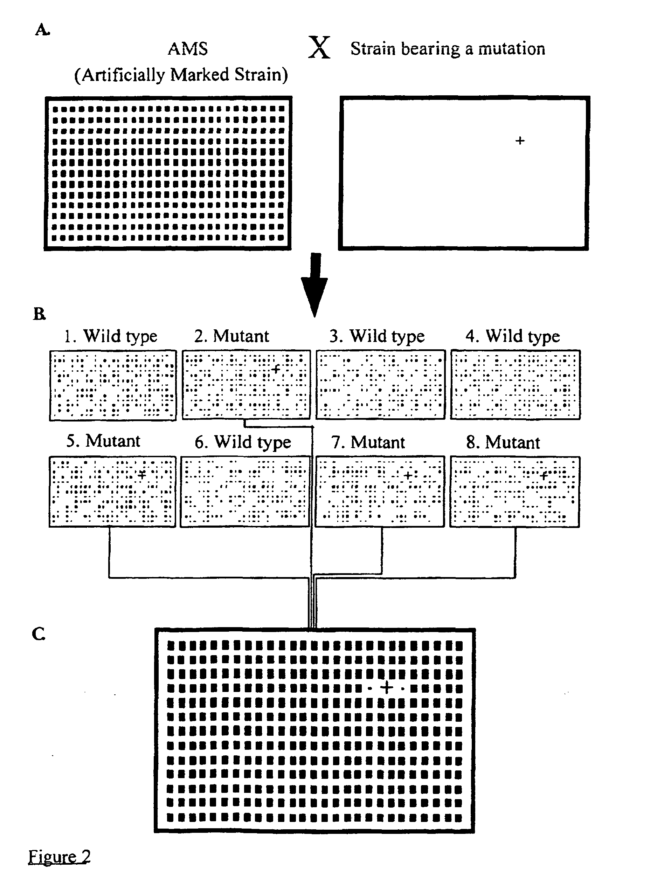

Figure 2 shows, according to an embodiment of the present invention, the mapping of mutations by means of the AMTEM™ technology. The artificially marked strain is crossed with another strain that differs in a certain property of interest due to one or more mutations (indicated by a "+") in the genome (A). The figure shows an example for one mutation. The other strain has no artificial markers. The segregants of the cross all have about 50% of the markers. The phenotype of the segregants is analysed to determine which of them have the property of interest. Since the property of interest is due to the mutation and since the mutation is derived from the strain without markers, all segregants with the mutant phenotype will lack the markers that are located closest to the position of the mutation. The markers can in principle be identified by checking each individual mutant segregant using micro-arrays containing the complement of all markers (B). It is more convenient, however, to pool the DNA of the segregants with the mutant phenotype and analyse the presence of all markers with the micro-array using the pooled DNA (C). All markers will be present except those that are located closest to the position of the mutation. Hence, the position of the mutation will be indicated by a sharp, transient drop in the intensity of the markers. The more mutant segregants are used the higher the resolution of the method, since the drop in signal intensity will be concentrated closer to the position of the mutation.

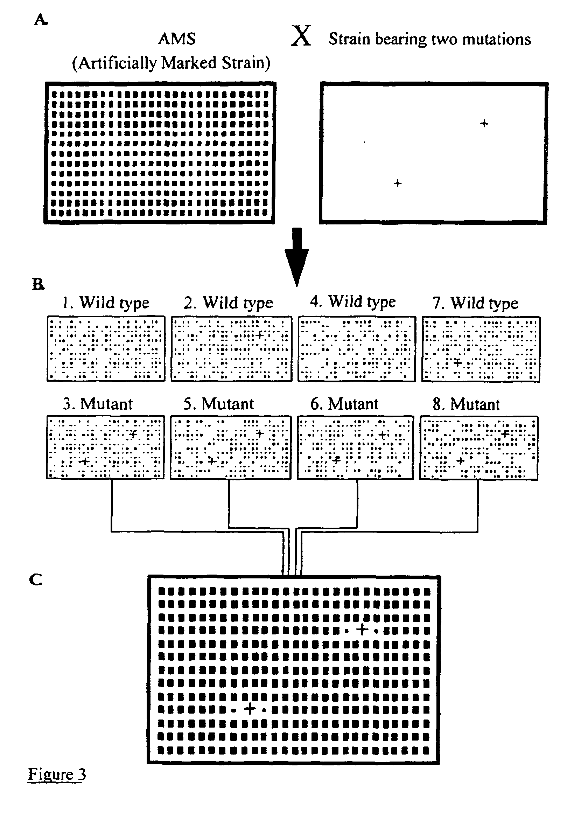

Figure 3 figure shows an example in accordance with an embodiment of the present invention where two mutations (indicated by a "+") are simultaneously required to cause the property of interest ('synthetic phenotype). Such a strain could be obtained after repetitive mutagenesis until a certain phenotype of interest is obtained. After crossing the marker strain with the mutant strain (A), the segregants are analysed for the presence of the mutant phenotype (B). Only segregants where the two mutations are present will show the mutant phenotype. The DNA of all segregants displaying the property of interest is pooled and the presence of all markers analysed with the micro-array (C). The position of both mutations will be simultaneously indicated by a drop in signal intensity of the markers located closest to the two mutations. The same principle applies when more mutations are simultaneously required for a specific phenotype. The position of each mutation will be indicated by a drop in signal intensity of the closest markers. Hence, in a single experiment the map position of all mutations required for a phenotype of interest can be determined.

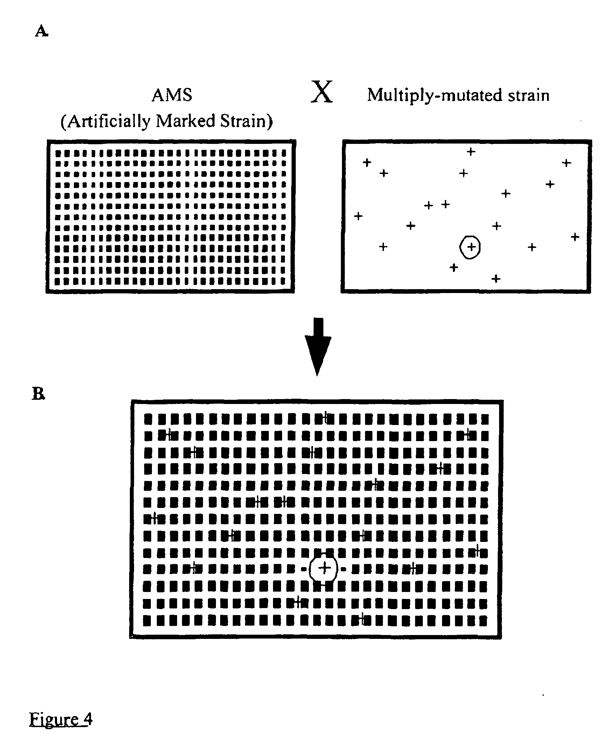

Figure 4 shows, according to an embodiment of the present invention, the use of a multiply-mutated strain to map the mutation that causes a particular phenotype. This figure and the next figure illustrate an important new development made possible by the AMTEM™ technology. In classical mutant isolation procedures, very many mutants were isolated with relatively light mutagenesis procedures. The purpose was to obtain strains with just one single mutation causing a specific, easily screenable phenotype. The disadvantage of this procedure is that only easily screenable phenotypes can be studied because of the large number of mutants that have to be analysed for the phenotype. the availability of AMTEM™ technology makes a new approach possible in which mutations are repeatedly accumulated within a single strain until a phenotype of interest is obtained. Since the phenotype of interest has to be checked only after multiple rounds of mutagenesis, phenotypes that are difficult to determine can also be studied. When the phenotype of interest is obtained, AMTEM™ technology can be used to map the position of all mutations required for the expression of this phenotype. The only requirement for this novel approach is that the mating capacity of the multiply-mutated strain remains high enough to cross it with the artificially marked strain (A) and that the diploid strain has sufficient sporulation capacity to obtain the required number of segregants. The DNA of the segregants with the phenotype of interest is pooled and the presence of the markers determined with the micro-array (B). The absence of markers reveals the position of the relevant mutation(s) (circled). All irrelevant mutations will segregate randomly and will therefore not influence identification of markers adjacent to the position of the relevant mutation.

It is understood that completely artificial markers are the preferred embodiment of the invention. In such a case the markers are created de novo. This does not exclude that the sequence of the markers can be present in other organisms, viruses, transposons, plasmids, etc. The only requirement is that the sequence is absent from the natural genome of the organism which is artificially marked. In another embodiment of the invention the markers could be composed in part of an artificial sequence introduced on purpose into the genome or naturally present in some isolates and in part of an adjacent sequence already present in the genome. In this case the artificial markers can be specific or they could all be identical and in the latter case the specificity can be provided by the adjacent sequence in the genome. As long as the two parts together constitute a specific marker, i.e. with a sequence that is nowhere present in the natural genome of the strain that is marked, the two parts together can be used as one single marker and constitute a specific artificial marker. In a further embodiment of the invention the artificial markers could be composed in part of natural sequences, such as those of transposons, viruses, plasmids, etc. which are on purpose introduced repeatedly into the genome to cover it genetically as much as possible and in part of the adjacent sequence already present in the genome. Also the 'scars' (short DNA sequences, also called footprints) which are left behind after transposition of a transposon (Plasterk R.H.A. and van Luenen H.G.A.M. 1997, In: Riddle D.L. et al., Eds. *C. elegans* II, Cold Spring Harbor Laboratory Press, Cold Spring Harbor, p.97-116) or after recombination of repeat sequences, such as the IoxP sequence which is recombined by the Cre recombinase (Sauer B. and Henderson N. 1990, New. Biol. 2, 441-449) can be used as artificial markers in conjunction with a specific sequence adjacent in the genome. As long as the two parts together constitute a specific marker, i.e. with a sequence that is nowhere present in the natural genome, the two parts together can be considered as one single artificial marker. Preferably, the natural sequences introduced on purpose in this way should be inserted in neutral positions in the genome. This can be done directly in case insertion occurs preferentially into intergenic regions. Or the natural sequences can be inserted randomly after which those that are inserted in neutral positions are identified by sequencing of the flanking DNA region and subsequently selectively accumulated into a single strain by repeated crossing. In a further embodiment of the invention the artificial part of the markers could be limited to one single nucleotide or to one or more nucleotide substitutions, while the other part is formed by an adjacent sequence already present in the genome. In conclusion, any technology making use of artificially introduced modifications of the genomic DNA sequence in order to create a set of specific markers for genetic mapping, and where the purpose is to cover the genome at least in part and preferentially as much as possible genetically with many such markers, falls within the scope of the present invention.
It is understood that the artificial markers can be introduced into the genome of the organism in different ways. Preferably, the artificial markers are introduced precisely at pre-determined positions using homologous recombination. This is described in Example 1. However, this is not essential for the invention and other strategies can be used. Markers can be inserted randomly into the genome by any one of a number of transformation methods. The DNA sequence at the insertion point is subsequently determined and only markers inserted in neutral positions are retained. The others are crossed out. Sequence analysis of the DNA region adjacent to an artificial marker can be done in the following way. The genomic DNA is fragmented with two restriction enzymes and an adaptor is ligated to one side of the restriction fragment. Subsequently, PCR amplification is performed with two primers, one of which is the artificial marker, the other in the adaptor. Alternatively, the genomic DNA is cut with one restriction enzyme, adaptors are added to both sides and vectorette PCR is used to amplify the fragment between the marker and the downstream adaptor. If more than one identical artificial marker was introduced, PCR amplified DNA fragments of different length will be obtained. The number of fragments will equal the number of identical artificial markers introduced into the genome. Sequence analysis of the PCR fragments will reveal the sequence of the insertion position of the artificial marker in the genome. If the complete genome sequence is known this will reveal the precise insertion point of the marker in the genome. It is understood that knowledge of the complete genome sequence is not truly essential but on the other hand greatly facilitates development of the AMTEM™ technology for a given organism. Currently, sequencing of complete eukaryotic genomes occurs at a very rapid pace. Examples are the sequencing of the genome of the yeast *Saccharomyces cerevisiae* (Mewes H.W. et al. 1997, Nature 387 Suppl.: 7-65), the nematode *Caenorhabditis elegans* (The C. *elegans* Sequencing Consortium 1998, Science 282, 2012-2018), the fruit fly *Drosophila melanogaster* (Adams M.D. et al. 2000, Science 287, 2185-2195), the plant *Arabidopsis thaliana* (The *Arabidopsis* Genome Initiative 2000, Nature 408, 796-815).
Knowledge of the complete genomic DNA sequence also facilitates the design of primers for the detection of the markers using PCR amplification (see further). Markers can also be introduced repeatedly using natural transposons, variants thereof or other DNA vehicles which insert at random positions into a genome. It is understood that a combination of the sequence or part of the sequence of such a natural DNA molecule, which is introduced on purpose many times into the genome so as to cover it genetically as much as possible, or which is already present as such in specific natural isolates, and part of the adjacent DNA is considered to represent also an 'artificial sequence' since it was not present before in the genome, at least not in most natural isolates, and was introduced or identified with the purpose of using it as a genetic marker. Hence, any technology using such genetic markers falls within the scope of the present AMTEM™ technology. Also the 'scars' (short DNA sequences, also called footprints) which are left behind after transposition of a transposon (Plasterk R.H.A. and van Luenen H.G.A.M. 1997, In: Riddle D.L. et al., Eds. *C. elegans* II, Cold Spring Harbor Laboratory Press, Cold Spring Harbor, p.97-116) or after recombination of repeat sequences, such as the loxP sequence which is recombined by the Cre recombinase (Sauer B. and Henderson N. 1990, New. Biol. 2, 441-449) can be used as artificial markers in conjunction with a specific sequence adjacent in the genome. They can be introduced through multiple transposition events or multiple recombination events of previously introduced repeat sequences. In principle, repeated mutagenesis causing nucleotide insertions, substitutions or deletions, followed by selection of strains with these mutations in neutral positions and combination of the mutation and adjacent sequence can also be used to create specific artificial markers and therefore also falls within the scope of the present invention. In conclusion any technology making use of DNA sequences introduced on purpose and repeatedly into a genome with the purpose of creating specific markers for genetic mapping falls within the scope of the present AMTEM™ technology.
It is understood that the pooling of the DNA of the segregants is not essential for the application of the new technology. As in most currently used genetic mapping technologies the genetic markers could also be scored in the segregants of the cross individually. However, because of the artificial nature of the markers their composition can be chosen so as to have a very high specificity compared to all other DNA sequences in the genome. As a result pooling of the DNA of all the relevant segregants does not interfere with the establishment of the genetic map and greatly facilitates the determination of the map positions, in particular for multigenic properties. How many segregants are required for establishment of the genetic map with the pooled DNA? One segregant contains statistically about 50% of the artificial markers, pooling of the DNA of two segregants results in about 75% of the markers, three in about 87,5%, four in about 93,75%, five in about 96,88%, six in about 98,44% and seven in about 99,22%. This indicates that with only seven segregants the chances of having all markers present in the pool are already very high.
It is understood that the strain with which the artificially marked strain is crossed can differ in one or in many properties. The only point that is relevant for the technology is that only those segregants that display the property of interest are pooled and their DNA used for the detection of the artificial markers.
It is understood that the strain with which the artificially marked strain is crossed is preferably isogenic [except for the mutations causing a particular phenotype of interest and the artificial (part of the) markers], less preferably heterogenic and still less preferably with an entirely different genetic composition. Since the segregants from the cross with this strain and the marked strain are selected on the basis of a specific trait, only the mutation or mutations important for the establishment of this trait will be preferentially selected and therefore only the markers adjacent to the position of these mutations will be absent. All other, i.e. non-relevant mutations will be distributed randomly in the segregants and therefore the markers adjacent to these mutations will also be distributed randomly and therefore show a similar signal intensity as all unlinked markers.

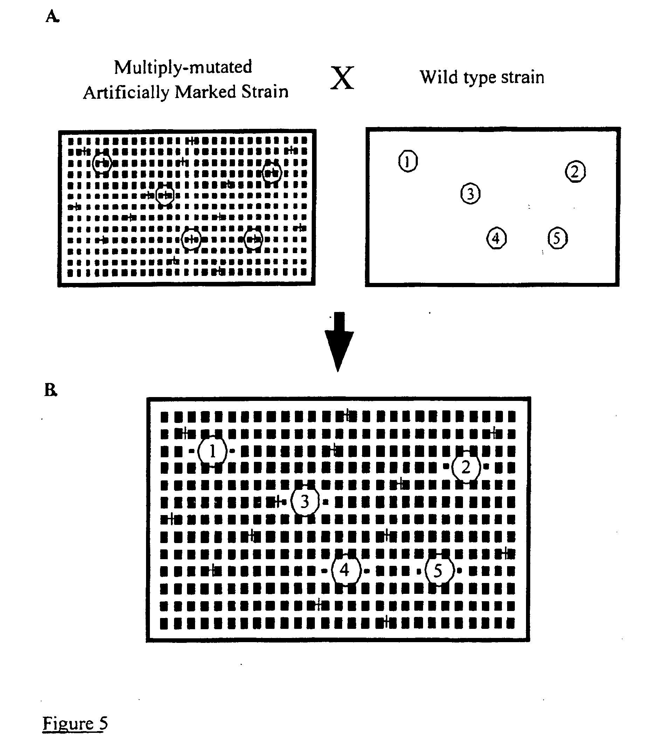

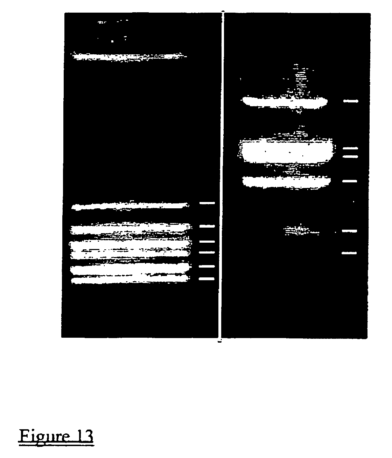

As a result, an interesting new development made possible by the AMTEM™ technology is that mutations can now be accumulated in one or a limited number of mutant strains rather than spread over as many strains as possible, as in classical mutant screens (illustrated in Fig.4 and 5). In the latter a strain is given a relatively light mutagenesis procedure to obtain many mutant strains with preferably only one mutation. A major disadvantage of this procedure is that only easy-screenable phenotypes can be studied. The AMTEM™ technology now allows to accumulate large numbers of mutations in a single or limited number of strains until a phenotype of interest is obtained. This phenotype can be difficult to determine, since it only has to be investigated in a limited number of strains. When a strain with the phenotype of interest is found, it is crossed with the artificially marked strain, the DNA of the segregants displaying the phenotype of interest is pooled and the presence of the markers determined. The position of all mutations relevant for the phenotype of interest will be indicated by a drop in signal intensity of the closest markers, whereas all irrelevant mutations will not influence the presence of the markers, since the irrelevant mutations are distributed randomly in the segregants (Fig. 4). In a further embodiment of the invention the strain in which the mutations are accumulated can be a strain in which multiple deletions of genes are accumulated. For instance deletions of genes can be made with high precision in yeast by homologous recombination. A collection of strains each with a deletion in one gene has been constructed (in case of essential genes a single deletion is made in a diploid strain resulting in a heterozygous strain) (Oliver S.G. et al. 1998, Trends Biotechnol. 16, 373-378; Winzeler E.A. 1999, Science 285, 901-906). At present single deletion strains for about 5800 of the estimated 6200 yeast genes are publicly available. These single gene deletions can be accumulated by multiple crossings in a small number of strains, so that a collection of multiple-deletion strains is available covering all or most of the genes. This collection can then be screened for a specific phenotype of interest. When the phenotype of interest is identified in one of the strains, the deletion(s) responsible for the phenotype can be identified rapidly by AMTEM™ technology. Example 6 (Fig. 13) describes the construction of a yeast strain with 12 deletions in its genome. The same methodology can be used to accumulate as many gene deletions in a single strain as possible without compromising viability and mating capacity, which are required for application of the AMTEM™ technology.
In a further embodiment of the invention, called Reverse AMTEM™, the artificially marked strain itself is mutagenised until a phenotype of interest is obtained. This can be done in many ways for instance by classical or transposon mutagenesis. In some organisms, such as yeast, it is possible to make precise gene deletions by homologous recombination. Such deletions can be introduced repeatedly in the artificially marked strain until a phenotype of interest is obtained. Moreover, collections of such multiply-mutated strains can be made in which as many mutations as possible are accumulated without abolishing the growth and mating capacity of the strain. These mutants will show many phenotypic changes, including a phenotypic change of interest. Such a mutant can now be used to determine the map position of all genes required for the expression of the wild type phenotype of interest. For that purpose the strain is crossed with a wild type strain of interest, which contains the wild type equivalent of all mutant genes including those causing either alone or jointly the phenotype of interest. The segregants are screened for the phenotype of interest and the DNA of all segregants that have regained the wild type phenotype of interest is pooled. The presence of the artificial markers is determined and all markers will be present except those that are located closest to the position of the individual mutations that are able to cause either alone or jointly the phenotypic change of interest. Hence, the map position of all genes required to express a specific wild type phenotype can be determined after a single cross. The principle of the Reverse AMTEM™ technology is explained with an example in Fig. 5. Herein depicted is an artificially marked strain which is mutagenised itself with multiple rounds of mutagenesis until a phenotype of interest is obtained, or it is saturation-mutagenised so that as many genes as possible are mutagenised without abolishing growth and mating capacity, or in organisms where this is possible (e.g. yeast), as many genes as possible are deleted by homologous recombination without abolishing growth and mating capacity. Several independent multiply-mutated marker strains can be developed. The multiply-mutated strain will show many phenotypic changes including a phenotypic change of interest. The latter is caused for instance by any one of five mutations present in the strain (circled "+"). Hence, every mutation alone can cause the phenotypic change by itself ('independent phenotype'). The multiply-mutated artificially marked strain is crossed with a wild type strain (A). It contains the wild type equivalents of all mutated genes, including those that can cause the phenotypic change of interest (numbers in circles).The segregants are screened for the phenotype of interest. Only segregants that have regained all five wild type genes (1 to 5) will have lost the phenotype of interest again and show the wild type phenotype. Micro-array detection of the markers obtained using the pooled DNA of these wild-type segregants reveals the position of all five genes that confer the phenotype of interest when individually mutated (B). Hence the map position of many or even all genes required to express a specific wild type phenotype is determined after a single cross.

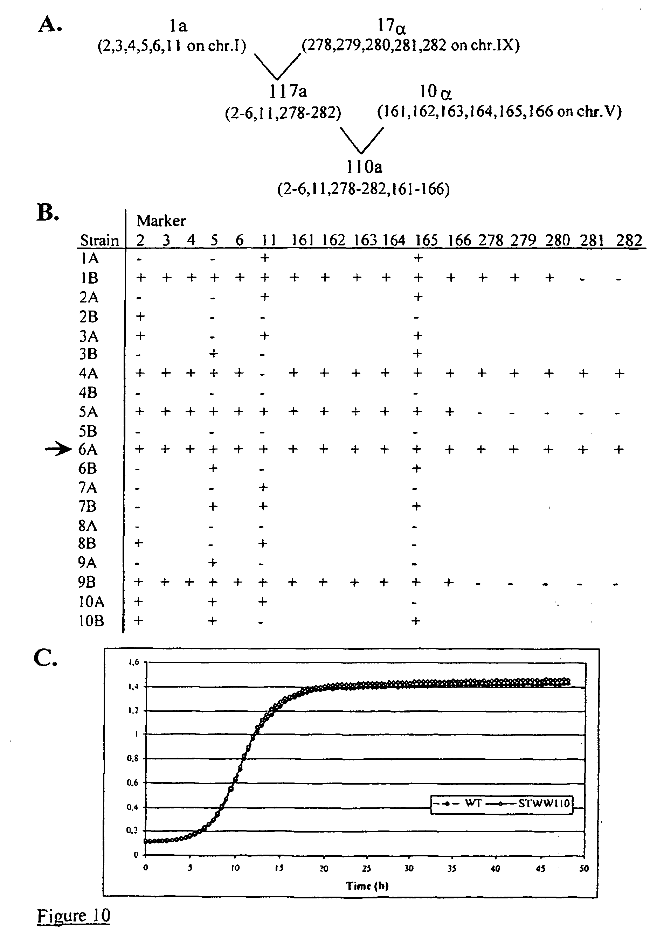

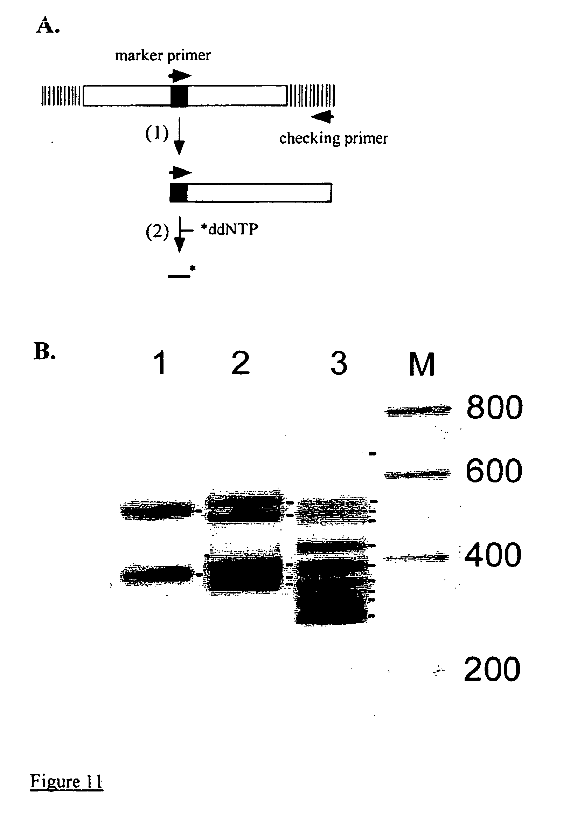

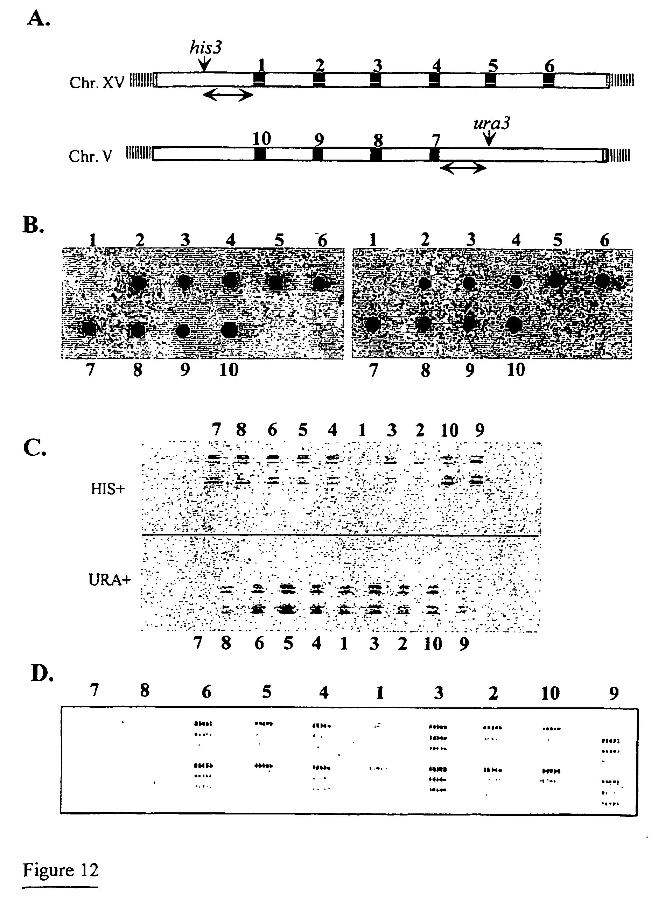

It is understood that the only requirement for successful genetic mapping with the AMTEM™ technology is the generation of viable, mating-competent segregants displaying the phenotype of interest. As a result, even a strain from a different species than the artificially marked strain could be used if it can be crossed successfully with the marked strain to generate viable first-generation descendants.
It is understood that also genes responsible for quantitative properties can be mapped with the AMTEM™ technology. If a strain differs in a quantitative trait from the artificially marked strain, the DNA of the segregants from the cross displaying the strongest difference with the artificially marked strain is pooled and the presence of the markers determined. The mutations responsible for the difference in the quantitative trait between the strain under study and the artificially marked strain will be preferentially selected and as a result the markers adjacent to the position of the mutations will be absent.
For the detection of the markers several existing methodologies are available. Whatever the methodology used, it is clear that the artificial character of the markers presents a unique advantage compared to all genetic mapping technologies which make use of natural genetic variation. Because the markers are artificial their composition can be chosen so as to allow the easiest and most sensitive detection. In particular the melting temperature of the artificial markers is made very similar which facilitates greatly the simultaneous detection of all the markers in a single experiment. Even if only part of the marker is artificial and the rest of the marker is a genomic sequence located adjacent to the marker, the length of this genomic sequence can be chosen such that the melting temperature of all markers is approximately the same, which greatly facilitates simultaneous detection of all markers. Such a detection method, which makes use of varying stretches of flanking sequence next to a marker to obtain a similar hybridisation temperature for all markers, has never been used before and is part of the present invention.
We describe a number of possible methods for the detection of the markers, which only serve as examples and are by no means exhaustive.
The detection method can for instance involve a PCR approach. For that purpose two oligonucleotide primers are used, one of which is identical to the marker and the other has a DNA sequence located at a convenient distance downstream in the genome. Interestingly, if this distance is taken somewhat different for different markers, many markers can be detected simultaneously in a single multiplex PCR reaction followed by gel electrophoresis (see Fig. 10,B and Fig. 12). Reaction products may be visualised by any of various DNA staining techniques, e.g. using SybrGreen or ethidiumbromide or using radioactivity or fluorescence or chemiluminescence. Alternatively real-time multiplex PCR may be used, in which case the amplification products are continuously detected during the PCR run, which avoids the electrophoresis step afterwards.
Another detection method involves a "double" PCR approach for the detection of the markers. This is illustrated in figure 11. Detection of artificial markers inserted in the genome. To facilitate the detection of the markers, the marker sequences are labeled, e.g. with a fluorescent or radioactive nucleotide (A). In one strategy, the genomic fragment containing the marker is first amplified by PCR (1), and a single base extension reaction in the presence of a labeled ddNTP (2) then produces a labeled marker sequence. Several markers may be combined in a multiplex PCR reaction, allowing the amplification (and subsequent labeling) of several marker sequences at once. In B, the 17 different marker-fragments present in the strain STWW110 are amplified in three multiplex PCR reactions, encompassing respectively 2, 5 and 10 primer pairs. The amplification products of different sizes are separated by agarose gel electrophoresis. Lane 1, marker 163 (373 basepairs) and 5 (511 basepairs); lane 2, marker 165 (345 bp), 164 (365 bp), 278 (384 bp), 281 (500 bp), and 3 (529 bp); lane 3, marker 162 (293 bp), 279 (314 bp), 6 (336 bp), 166 (354 bp), 11 (384 bp), 4 (421 bp), 282 (481 bp), 2 (499 bp), 280 (525 bp), and 161 (648 bp); M, standard DNA size markers (numbers denote the basepairs).
Following multiplex PCR reaction, a multiplex Single Base Extension (SBE) or Multi Base Extension (MBE) reaction is performed using the multiplex PCR reaction product as the template and oligonucleotides identical to the markers to be detected as primers (illustrated in Fig. 11,A). In the SBE reaction, only radioactive or fluorescently labeled ddNTP is used. In the case of MBE reaction, one of the four dNTPs is labeled with a radioactiveor fluorescent compound. Either with or without purification, the product of the SBE or MBE reaction can then be hybridised to the reverse complements of the markers (for SBE reaction) or longer oligonucleotides containing the reverse complements of the markers (for MBE reaction) spotted on a nylon membrane or in a high-density micro-array format on a glass slide (see Fig. 12). This provides a high throughput format for simultaneous detection of hundreds to thousands of markers in a single assay.
The detection can also be done without the involvement of a PCR approach. In this case, the fragmented genomic DNA is hybridised directly to the array and the SBE or MBE reaction is performed on the array, or the SBE/MBE reaction is done first with fragmented genomic DNA as template and then the reaction mixture is hybridised to the array with or without purification.
In all cases described here, the final detection of a marker is based on the presence of the radioactive or the fluorescent signal that is specifically associated with this marker. Radioactivity can be detected using phosphorimager technology while fluorescence can be detected using a fluorescence scanner or CCD camera.

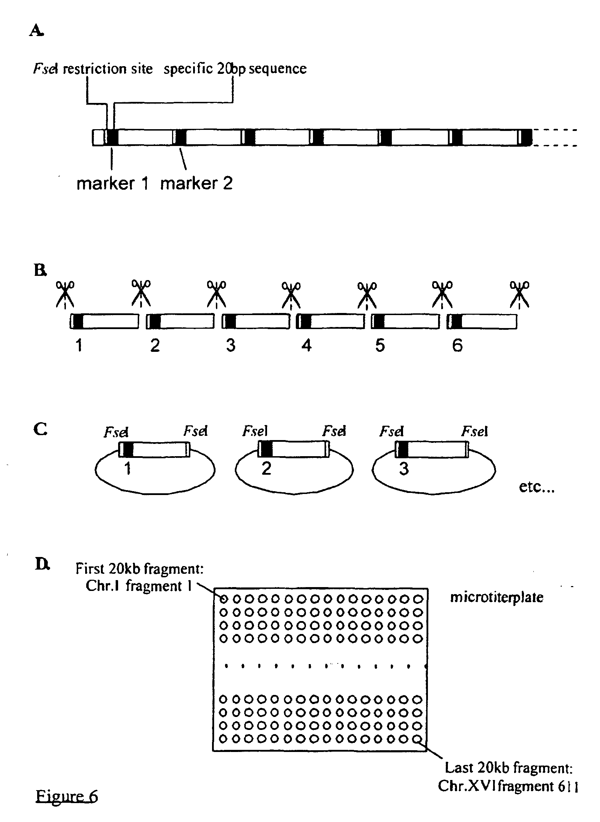

We show that the introduction of artificial markers in the genome creates unexpected and entirely novel opportunities. In a further embodiment of the invention, a restriction site for a rare-cutting enzyme can be added adjacent to the marker (Fig.6). Preferably, the restriction enzyme should not cut anywhere in the genome. This can be determined using the complete genome sequence of the organism or tested experimentally. If the restriction enzyme cuts in a few places in the genome the restriction sites can be eliminated by standard site-directed mutagenesis procedures. The presence of the restriction site in the artificial marker allows to cut the genomic DNA with the restriction enzyme in a limited number of fragments which each contain a specific marker. The fragments are then cloned into a vector and after transformation of the resulting constructs into a host cell, such as *Escherichia coli* or bacteriophage lambda (λ), the inserts can be identified on the basis of the marker. The transformants are then sorted in the proper order (from the first fragment of the first chromosome to the last fragment of the last chromosome) for instance in wells of microtiter plates. All constructs are also mixed together to make a complete genomic library. The quality of the library (presence of all genomic fragments) can always be assessed easily, for instance using the micro-array detection method for the artificial markers. After transformation of the genomic library into a recipient strain and plating out the transformants, duplicates of the transformants are pooled together and complete coverage of the whole genome in the transformants can be checked on the basis of the presence of the specific artificial markers. Such a check for complete coverage of the genome in the transformants has never been possible before with a genomic library. Any genome fragment that is lacking in the pool of transformants can be taken from the sorted library in the microtiter plate and transformed individually into the recipient strain. This novel procedure guarantees complete coverage of the genome in a limited number of transformants. It also allows to reduce the number of transformants required for complete coverage considerably since any clones that are lacking can afterwards be introduced individually. In all existing transformation procedures with genomic libraries there is never certainty that the whole genome is covered and the number of transformants required has to be many times the estimated number of genomic inserts in the library in order to maximize the chances that the whole genome is covered.
As mentioned previously the ideal genetic mapping technology should have markers that are entirely independent of the phenotype of the organism. This is the case with the AMTEM™ technology where the markers are artificial oligonucleotides inserted on purpose in neutral positions in the genome. Depending on the organism used the artificial markers can also be inserted randomly in the genome after which appropriate markers can be identified by sequence analysis of their insertion position and accumulated by crossing into a single organism. Because of the use of artificial markers the AMTEM™ technology allows otherwise isogenic strains to be used for isolation of mutants and subsequent mapping. In the AMTEM™ technology the markers are either inserted in specific positions so as to be evenly spaced over the whole genome or they are selected to cover the whole genome. The appropriate number of markers is determined based on the recombination frequency of the organism. It is taken high enough to detect linkage with any gene and not higher than necessary to avoid collection of useless information. The artificial AMTEM™ markers can be detected simultaneously for instance with a high-density micro-array or with multiplex PCR. To facilitate the mapping of the mutation(s) of interest, the DNA of all the segregants displaying a specific phenotypic trait can be pooled and the presence of the markers determined for all segregants together with one single experiment. As a consequence, the genetic map position of all mutations required for a particular trait can be determined with just one genetic cross. This greatly simplifies the mapping procedure, in particular for multigenic properties. Hence, because of the use of a large number of artificial markers, genetically covering the genome completely, the AMTEM™ technology is the first mapping technology that comes close to the ideal genetic mapping method.

### Examples

The AMTEM™ technology can be performed with any eukaryotic organism capable of sexual reproduction. In principle an artificially marked strain can be constructed for any such organism. The only difference is the effort and time required for the construction. With some organisms the marker oligonucleotides can be inserted precisely on predetermined positions in the genome. In this way insertion of markers in positions affecting the phenotype can be avoided as much as possible on beforehand, which saves time for the construction of the strain. This is the best and therefore preferred method. However, it is also the most difficult method. As an example of the embodiment of the invention we describe the construction of an artificially marked strain of the yeast *Saccharomyces cerevisiae*. With other organisms the marker oligonucleotides are simply inserted at random in the genome with anyone of several methods available. Subsequently, their position is determined by sequencing of the adjacent region in the genome and only the markers that are inserted in neutral positions and therefore unlikely to have effect on the phenotype are retained. All markers of interest are accumulated in a single strain by crossing. Markers inserted in undesirable positions are crossed out.

### Example 1 Construction of an artificially marked strain of the yeast Saccharomyces cerevisiae.

We demonstrate how an artificially marked strain of the yeast *Saccharomyces cerevisiae* can be constructed. First we have calculated on the basis of known recombination percentages from the literature how many markers are approximately needed to cover the whole genome genetically.
Using classical mapping data from the literature combined with the data on the distribution of the yeast ORF's from the sequencing programme we have made a prediction of the decrease in intensity of a signal derived from an artificial marker located at a certain distance from a specific mutation compared to a signal derived from an unlinked marker. In the following examples we can each time assume for comparison that one of the mutations is a mutation of interest and the other one is the closest artificial marker. If there is no linkage 50 % of the descendants will have the marker. Hence, we have to compare the number of double mutants obtained with 50% of the total number of descendants. A number of examples of such genetic crosses is shown below. (PD='parental ditype'; NPD='non-parental ditype'; T='tetratype')
1. SSN2-SNF1
   Distance: 14,4 cM
   Number of genes in between: 41 genes
   Mapping data: PD: 15 NPD: 0 T: 6
   Total number of segregants: 84; 50% = 42
   ssn2 snf1 segregants: 6
   Intensity of marker: 6/42 or 1/7 of the intensity of the unlinked markers
2. CYR1-ILV3
   Distance: 10 cM
   Number of genes in between: 22 genes
   Mapping data: PD: 16 NPD: 0 T: 4
   Total number of segregants: 80; 50% = 40
   cyr1 ilv3 segregants: 4
   Intensity of marker: 4/40 or 1/10 of the intensity of the unlinked markers
3. HIS3-STE13
   Distance: 29,9 cM
   Number of genes in between: 16 genes
   Mapping data: PD: 11 NPD: 0 T: 15
   Total number of segregants: 104; 50% = 52
   his3 ste13 segregants: 15
   Intensity of marker: 15/52 or ±1/3.5 of the intensity of the unlinked markers
4. ADE2-SUF5
   Distance: 9,9 cM
   Number of genes in between: 14 genes
   Mapping data: PD: 145 NPD: 1 T: 29
   Total number of segregants: 700; 50% = 350
   ade2 SUF5 segregants: 29
   Intensity of marker: 29/350 or ±1/12 of the intensity of the unlinked markers
5. URA3-GCN4
   Distance: 7,5 cM/9,6 cM/8,4 cM
   Number of genes in between: 12 genes
   Mapping data: PD: 52/139/45 NPD: 0/0/0 T: 9/32/9
   Total number of segregants: 1144; 50% = 572
   ura3 gcn4 segregants: 50
   Intensity of marker: 50/572 or ±1/11 of the intensity of the unlinked markers
6. URA3-MCM3
   Distance: 8,3 cM
   Number of genes in between: 10 genes
   Mapping data: PD: 5 NPD: 0 T: 1
   Total number of segregants: 24; 50% = 12
   ura3 mcm3 segregants: 1
   Intensity of marker: 1/12 of the intensity of the unlinked markers
7. HIS3-STE4
   Distance: 11,8 cM
   Number of genes in between: 9 genes
   Mapping data: PD: 10 NPD: 0 T: 3
   Total number of segregants: 52; 50% = 26
   his3 ste4 segregants: 3
   Intensity of marker: 3/26 or ± 1/9 of the intensity of the unlinked markers
8. SNF1-RNA3(PRP3)
   Distance: 5.6 cM
   Number of genes in between: 3 genes
   Mapping data: PD: 87 NPD: 0 T: 11
   Total number of segregants: 392; 50% = 196
   snf1 ma3 segregants: 11
   Intensity of marker: 11/196 or ± 1/18 of the intensity of the unlinked markers
9. ADE2-PFY1
   Distance: too short to determine
   Number of genes in between: 5 genes
   Mapping data: PD: 38/7 NPD: 0/0 T: 0/0
   Total number of segregants: 180; 50% = 90
   ade2 pfy1 segregants: 0
   Intensity of marker: 0, the marker will disappear completely

The relationship between the mapped distance (cM) and physical distance (kb, reflected in the number of genes in between, in average there are about 10 genes per 20 kb in the yeast genome) is not always the same because of the differences in recombination frequency over the length of the chromosomes.

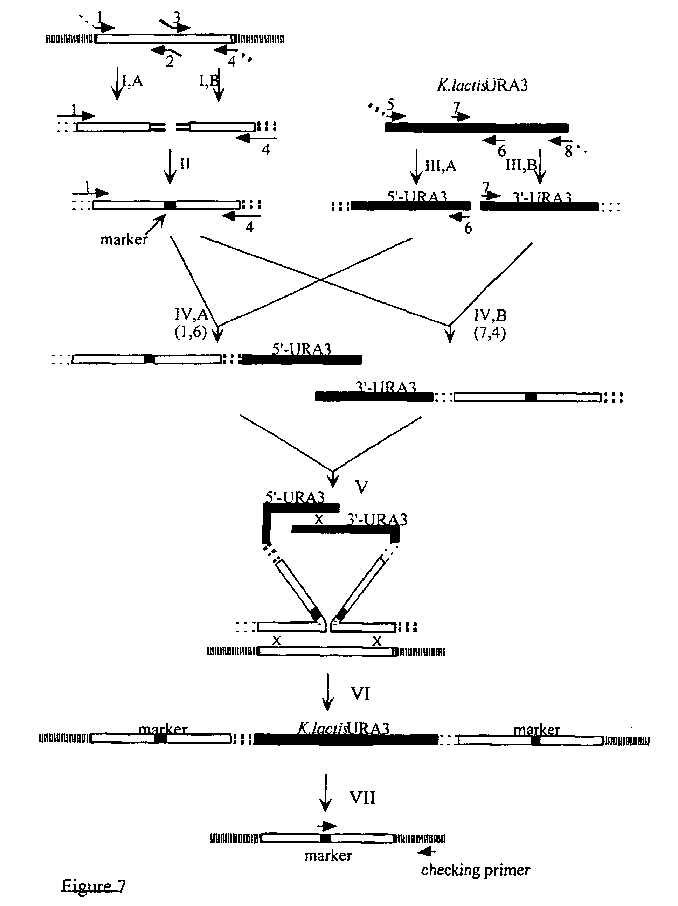

This is the reason why in the above examples the genetic distance covers different numbers of genes. In general however this relationship is quite constant. Whatever the precise relationship between map distance and physical distance, these examples clearly show that when the distance between the artificial marker and the mutation under study would be 10 genes, the signal intensity of the marker will drop to about 10% of the other (unlinked) bands. This should be clearly visible. In the case of a distance of 5 genes, the signal intensity of the marker will generally be zero. This means that if we insert a marker every 10 genes and the mutation is located in the middle between two markers, both markers will most probably disappear completely from the track of markers. Of course, if the mutation is not located in the middle, the closest marker will certainly disappear completely. These results indicate that a spacing of about 10 genes (or about 20 kb) in between two markers should be appropriate for our purposes.
Hence, the total number of markers has been chosen in such a way that after the selection of the segregants with the phenotype of interest and the pooling of their DNA, at least one marker should disappear completely, whatever the location of the responsible mutation in the genome. Given the variation of the recombination frequency over the genome and the higher reliability of at least two markers disappearing adjacent to the mutation (one on each side) the preferred number of markers needed was determined at about 600.
Subsequently, the markers were distributed with intervals of 20 kb over the different chromosomes in the genome of the strain S288C. The first marker was positioned upstream of the first gene on chromosome I and the last marker downstream of the last gene on chromosome XVI. With this distribution the total number of markers is 611. This distribution of the markers is shown in Fig. 1,B. It is understood that this number is only an example and that any other number that covers part of the genome genetically and preferentially covers it completely is appropriate.
The strain to be used can be any strain of the yeast *Saccharomyces cerevisiae*, but preferentially the strain S288C of which the whole genomic DNA sequence has been determined. This facilitates determination of the precise insertion points for the markers since the position of the genes is precisely known and also the DNA sequence of the insertion points is precisely known. It allows selecting highly specific markers; i.e. markers of which the sequence is entirely absent from the whole genomic DNA sequence. The sequence of the markers has been determined using public DNA sequence databases from other organisms and manual modification of the sequence. Aii markers have been checked for absence of sequence similarity with any sequence in the total yeast genome sequence.
For the insertion of every marker, six specific primers are used and four universal primers are used for the amplification of the *K. lactis* URA3 gene.
Five examples of artificial marker sequences that have been inserted in a yeast strain are shown below: The following PCR Primers were used for the introduction of these markers:
Primers 1 and 2 are used for PCR I,A;primers 3 and 4 are used for PCR I,B; primers 1 and 4 are used for PCR II; the checking primer (CHE), in combination with the marker primer, is used to check proper introduction of the marker in the genome.
The two adaptor sequences attached to primer 1 and 4 respectively are: These adaptor sequences are complementary to the adaptor sequences attached to primer 8 and 5 respectively, which are used in the amplification of respectively the 3'- and 5'-end of the *K. lactis* URA3 gene (see Fig. 7). The adaptor sequences attached to primer 2 and 3 are complementary to each other and correspond to the marker sequence in a reverse (antisense) and forward (sense) orientation respectively. All adaptor sequences are shown in italics below. An *Fse*I restriction site is added to the adaptor sequences in primers 2 and 3 (underlined below), which results in the addition of an Fsel site at the 5'-end of the inserted marker (see Example 2).

Figure 7 shows an overview of the strategy for the insertion of artificial markers in the yeast genome. The 5'- and 3'-part (I,A and I,B respectively) of the genomic sequence flanking the desired marker insertion site are amplified by PCR using primers (1 to 4) containing different adaptor sequences. Remark that the adaptor sequences on primer 1 and 8, those on primer 4 and 5, and those on primer 2 and 3, are pairwise complementary to each other. Both PCR fragments are linked again in a subsequent PCR step (II), thereby inserting the marker sequence, which corresponds to the adaptor sequence on primer 2 and 3. The amplification product obtained is similarly linked with the 5'-part or with the 3'-part of the *K. lactis* URA3 gene in PCR step IV,A and IV,B respectively. The partially overlapping *K. lactis* 5'- and 3'-parts are obtained in PCR step III,A and III,B respectively. The resulting chimeric PCR products, containing either part of the *K. lactis* gene and an identical genomic sequence flanking the artificial marker sequence, are combined (V), transformed into an *ura3* strain and the transformants grown on URA- medium, lacking uracil (VI). Only the transformants that have integrated the *K. lactis* URA3 gene fragments in the correct way, thereby forming an active URA3 gene, are able to grow on the URA- medium. As a result of the homologous recombination events, most of the transformants now have two copies of the marker sequence. When these transformants are subsequently grown on FOA selection medium, which is toxic for cells expressing the URA3 gene, pop-out of the URA3 gene through homologous recombination eliminates the URA3 gene from the genome and yields a strain with a single marker sequence inserted at the desired genomic location. The presence of the marker at the correct genomic location is verified by PCR amplification using a primer complementary to the marker sequence and a "checking primer" complementary to a genomic sequence downstream of the flanking sequence used for homologous recombination of the marker-containing PCR fragment (arrows). The same strategy is repeated for every marker that is inserted in the yeast genome until the whole genome is covered with artificial markers.

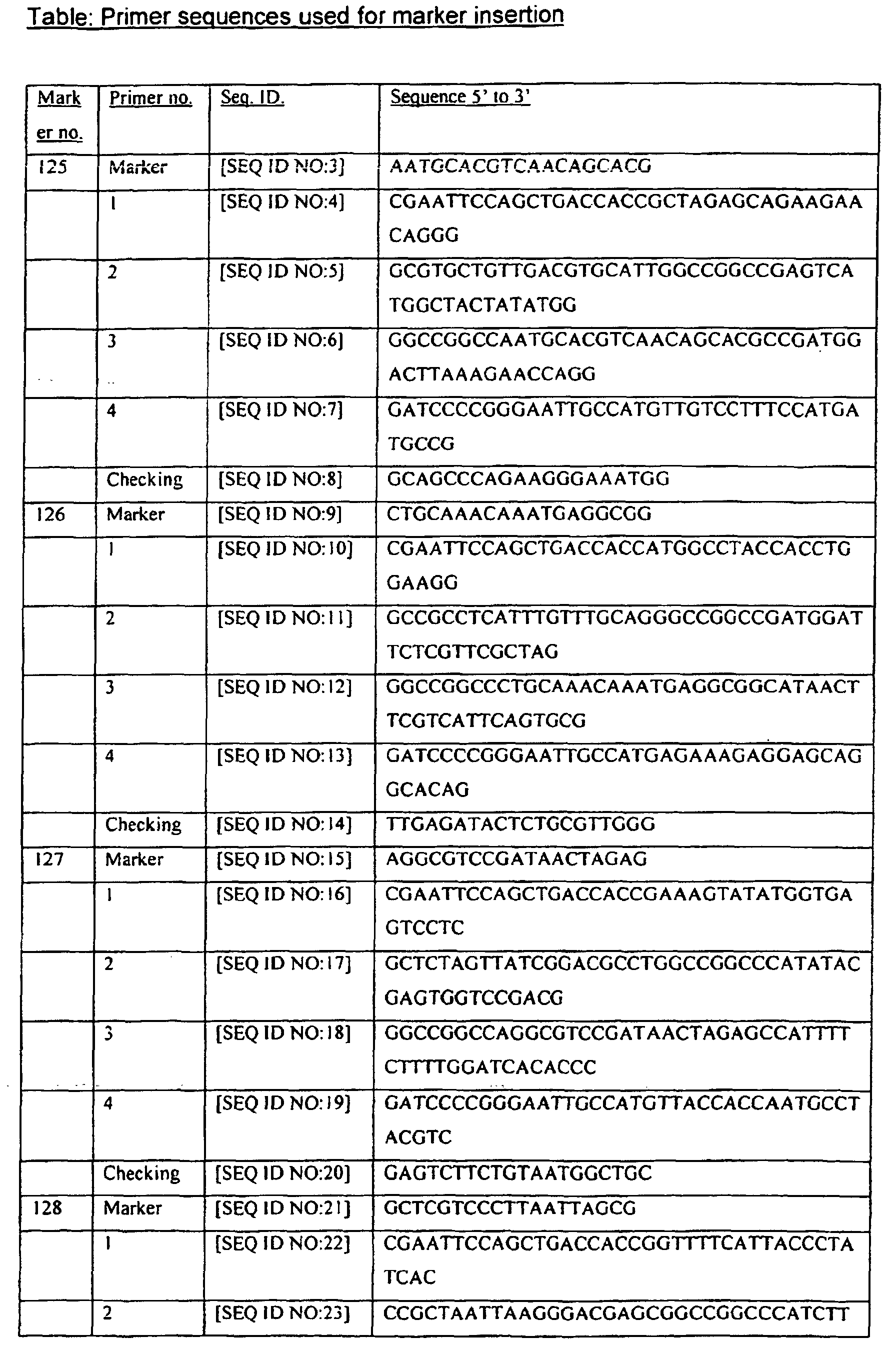

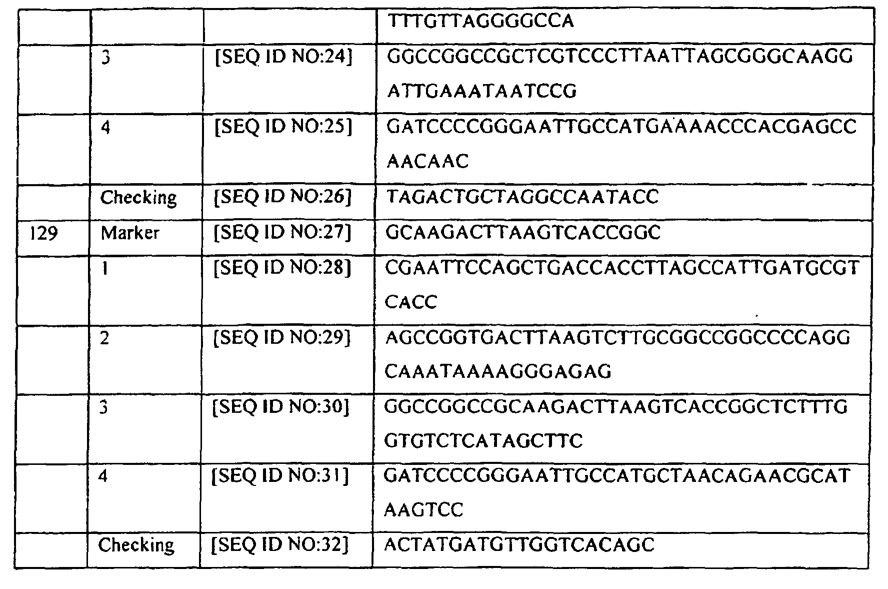

The sequences of the four universal primers used for amplification of the *K. lactis* URA3 gene are as follows (adaptor sequence in italics): The markers have been inserted using the strategy shown in Fig. 7. After determination of the genomic insertion point, two pairs of PCR primers were designed. Each PCR primer consisted of two parts. The first primer (primer 1, forward orientation) consisted of adaptor A as part 1 and a genomic sequence about 200-300 bp upstream of the insertion point, as part 2. Its companion primer (primer 2, reverse orientation) consisted of the marker, as part 1 and the genomic sequence flanking the insertion point downstream on the other strand, as part 2. These two primers are used for PCR reaction I,A with genomic DNA of strain S288C as template. Primer 3 (forward) consisted of the marker, as part 1 and the genomic sequence flanking the insertion point downstream, as part 2. Its companion primer (primer 4, reverse) consisted of adaptor B as part 1 and a genomic sequence about 200-300 bp upstream of the insertion point on the other DNA strand, as part 2. These two primers are used for PCR reaction I,B with genomic DNA of strain S288C as template. Subsequently, PCR reaction II is performed with primers 1 and 4 and using the products of PCR reactions I,A and I,B as mixed templates. This generates a DNA fragment that contains a part of the genomic DNA sequence with the marker inserted at the correct position and with adaptor A and adaptor B as flanking sequences. Subsequently, two overlapping parts of the *Kluyveromyces lactis URA3* gene were amplified by PCR (PCR III,A and III,B). For PCR III,A the following primers were used. The first primer (primer 5, forward) consisted of adaptor b, which is complimentary with adaptor B, and the most upstream fragment of the *URA3* gene. The second primer (primer 6, reverse) consisted of a sequence just over the middle downstream in the *URA3* gene. For PCR III,B the following primers were used. The first primer (primer 7, forward) consisted of a sequence just over the middle upstream in the *URA3* gene. The second primer (primer 8, reverse) consisted of adaptor a, which is complimentary with adaptor A, and the most downstream sequence of the *URA3* gene. Subsequently the products of PCR reaction II and III,A were used as template for PCR reaction IV,A with the primers 1 and 6. The products of PCR reaction II and III,B were used as template for PCR reaction IV,B with the primers 7 and 4. The products of PCR reactions IV, A and IV,B were then co-transformed as such into the strain S288C which contains a *ura3* mutation and is therefore auxotrophic for uracil (V). Uracil prototrophic transformants were then selected on a medium lacking uracil. To gain prototrophy for uracil the cells have to integrate the two constructs in the genome so that a functional *URA3* gene is restored. As an added result this *URA3* gene is flanked on both sides by a copy of the genomic DNA sequence with the marker inserted (VI). The strain is subsequently transferred to medium containing 5-fluoroorotic acid (FOA). On such a medium *URA3* prototrophic strains are unable to grow and only strains that have eliminated the *URA3* gene by recombination between the two repeat sequences of genomic DNA with the marker will be able to grow (VII). Such strains have the marker inserted at the correct position in the genome. This procedure can now be repeated for the second marker, the third marker, etc. until all the markers have been introduced in the correct position in the genome.

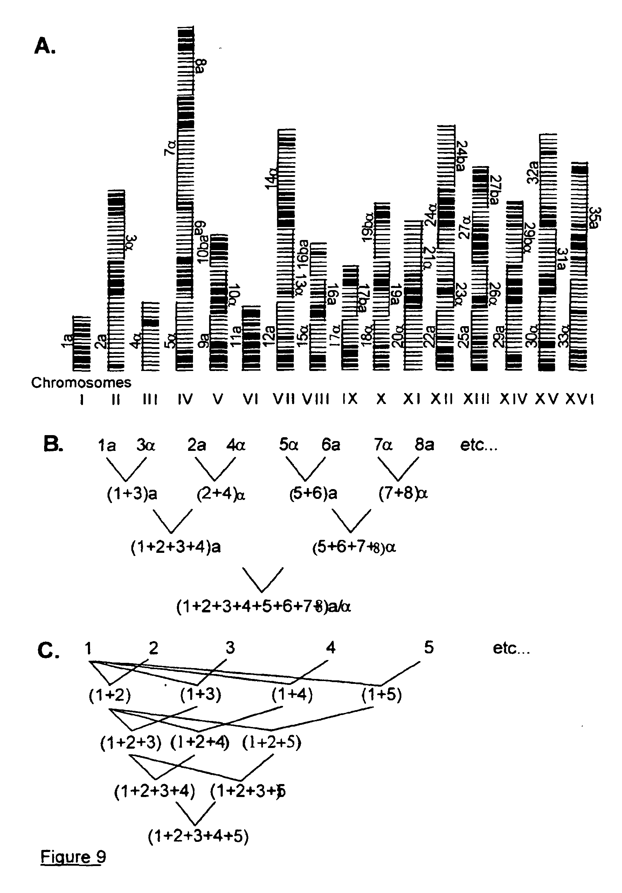

A strain developed with this methodology and carrying the five artificial markers mentioned (numbers 125 to 129 in strain 7α on figure 9,A) (STWW125-129) has been deposited by Dr. Johan Thevelein, Dr. P. Ma and Dr. Patrick van Dijck (all from the Laboratory of Molecular Cell Biology, K.U.Leuven, Institute of Botany and Microbiology, Kasteelpark Arenberg 31, B-3001 Heverlee Belgium) with the Belgian Coordinated Collection of Microorganisms (BCCM) Scientific institute of Public Health- Louis Pasteur-Mycology IHEM (J. Wytsmanstraat 14, B 1050 Brussels, Belgium) on June 21, 2001 under the Accession Number IHEM 18728.
A strain containing seventeen (17) artificial markers has also been deposited (called strain STWW110) and is the result of 2 subsequent crosses involving three strains that have been developed using the marker insertion methodology described (see below).

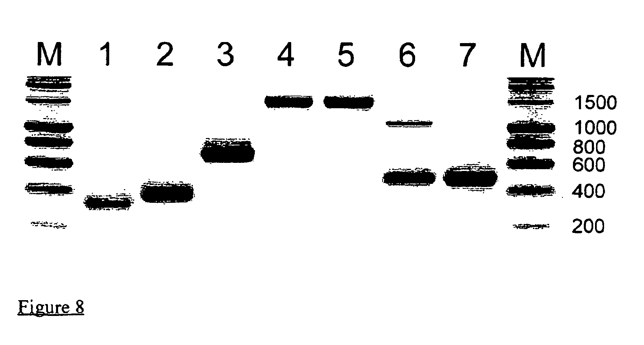

In Figure 8, the outcome of the PCR analysis in every step of the marker insertion strategy is shown for one of the markers that were introduced in the yeast genome (marker no. 168, introduced in strain STWW110 (see below) which now contains eighteen markers).

In figure 8 the amplification products obtained at each step during the insertion of marker 168 are shown. Lane 1, PCR reaction I,A; lane 2, reaction I,B; lane 3, reaction II; lane 4, reaction III,A; lane 5, reaction III,B; lane 6, reaction IV,A; lane 7, reaction IV,B; lane 8, PCR using the marker and checking primers and primer 6 for the *K. lactis* URA3 gene and as a template a URA "min" (URA-) colony obtained following the transformation of yeast with the PCR products of reaction IV,A and IV,B; the large DNA band (approximately 1,2 kb) extends from the left marker copy to the recombined URA3 gene and the small DNA band (474 bp) extends from the right marker copy to the checking sequence in the genome; the presence of both bands proofs that the insertion occurred according to the recombination scheme shown in figure 7; lane 9, final proof of marker insertion using the marker and checking primers and DNA from a colony obtained on FOA selection plates as a template; the presence of the 474 bp fragment indicates that the artificial marker was inserted at the correct position in the yeast genome.

The following primers were used for the insertion of marker 168:

By using primers containing adaptor sequences, the amplification products obtained are tagged with artificial sequences that may serve as primers in subsequent PCR reactions. In PCR step II, this results in the insertion of the artificial marker sequence, while in PCR step IV, this results in the generation of a chimeric sequence consisting of a genomic fragment containing the marker and either part of the *K.lactis* URA3 gene. The integration of the chimeric URA3 sequences into the yeast genome is checked by PCR with 3 primers (marker and checking primer and universal primer 6), confirming the presence of two copies of the marker-containing genomic fragment, which are separated by the recombined URA3 gene (see Fig. 7). The use of a checking primer that binds to a sequence outside the fragment used for transformation further confirms that the fragment was inserted at the correct genomic site. When the URA- colonies are transferred to FOA medium, only those cells that have removed the URA3 gene through homologous recombination are able to grow; the pop-out of the URA3 gene results in strains that have acquired a single copy of the marker in the genome.
A test strain with six consecutive markers introduced at 20 kb intervals from the *his3* locus and four consecutive markers introduced at 20 kb intervals from the *ura3* locus has also been constructed with this methodology to demonstrate the proof of principle of the AMTEM™ technology (see examples 4 and 5).

It is understood that the insertion of the markers is a simple repetitive process and that in principle all markers can be inserted one by one in the same strain to generate the strain with the 611 markers. However, in a preferred embodiment of the invention an additional strategy can be used to fasten the introduction of the markers. In this strategy the markers are not introduced one by one in a single strain, but they are introduced concurrently in different strains after which the strains are crossed with each other to accumulate all the markers in the same strain.
An example of such a strategy is shown in Fig. 9. Markers are introduced into 40 strains. The 13 markers of chromosome I are introduced into a single strain, no.1, starting with marker no. 1. The 54 markers of chromosome II are introduced into two strains, no.2 and no.3. The markers of the other chromosomes are introduced in the same way in the remaining 37 strains. The location and number of markers in each strain are indicated in Fig. 9,A. In this figure, the markers that have been introduced successfully are shown in bold.
To facilitate the accumulation of all markers of chromosome II afterwards into a single strain, one could make use of an overlap of for instance 5 markers. Hence, strain no.2 would contain the marker 14 to 36 and strain no.3 the markers 32 to 54. These two strains can be constructed separately. However, to speed up the construction of the strain the preferred embodiment of the invention is to introduce first the 5 markers of the overlap into one strain and then to duplicate this strain into two strains in which markers are inserted in an opposite direction. This approach has e.g. been used to generate strains 20α and 21α, which have a 5-marker overlap region. Alternatively, the original 5-marker strain may be used to generate by any one of several available procedures the same strain with the opposite mating type. This can be achieved by crossing of the strain with a wild type strain or transformation with a plasmid containing the HO gene which results in frequent mating type switching. In a more preferred embodiment of the invention the strain with the 5 markers is crossed with another strain containing a 5-marker overlap region of another chromosome (or from the same chromosome in case there is more than one overlap region on a chromosome). From this cross segregants are taken of the two mating types that contain both overlap regions of 5 markers. This procedure facilitates the later accumulation of all markers into a single strain.

After introduction of the markers into the 40 strains, the strains are crossed with each other to accumulate all the markers into one single strain. Different strategies can be used for this purpose. For instance, strain no.1 can be crossed with strains 2 to 40 in order to obtain 39 strains which all have the markers of chromosome no. I in addition to the specific markers of another chromosome (1+2, 1+3, etc.). Subsequently, strain no.(1+2) is crossed with strains (1+3) to (1+40) to accumulate the markers of strain 2 in all the others. The new strains now have the markers of strain 1, strain 2 and their original markers. This strategy is outlined in Fig. 9,C. Another strategy, outlined in Fig. 9,B, is to cross strain 1 with strain 2, strain 3 with strain 4, etc. so as to reduce the number of strain with 50% and double the number of markers in each strain. Subsequently, the strains are again crossed two by two to halve the number of strains and double the number of markers per strain. A mixture of these two strategies can also be used. One of the additional strategies to facilitate this crossing strategy is to insert markers in partly overlapping genomic regions such that two partly overlapping strains are obtained that mark the same chromosome. This overlapping region may e.g. correspond to five adjacent markers as was explained before.
It is understood that the precise way in which the markers are accumulated into a single strain is not essential for the invention. This can be done in many ways. Even during the introduction of the markers, strains can already be crossed with each other so as to reduce the number of strains and increase the number of markers per strain. Also if the accumulation of all markers by crossing proves to be difficult for the last markers, the lacking markers can be introduced directly with the strategy shown in Fig.7.
A yeast strain (named STWW110) containing seventeen (17) artificial markers in the genome has been deposited by Dr. Johan Thevelein, Dr. Wim Broothaerts, Dr. Françoise Dumortier and Dr. Patrick van Dijck (all from the Laboratory of Molecular Cell Biology, K.U.Leuven, Institute of Botany and Microbiology, Kasteelpark Arenberg 31, B-3001 Heverlee Belgium) for, Prof.

Koenraad Debackere and Dr. Paul Van Dun, representatives of the legal entity, K.U.Leuven Research & Development (Groot Begijnhof 59, B-3000 Leuven, Belgium) with the Belgian Coordinated Collection of Microorganisms (BCCM) Scientific institute of Public Health- Louis Pasteur-Mycology IHEM (J. Wytsmanstraat 14, B 1050 Brussels, Belgium) on June 14, 2002 under the accession number IHEM 19413. This strain was obtained by the crossing strategy shown in Fig. 10,A. Strain 1a containing 6 markers on chromosome I was crossed with strain 17α which contains 5 markers on chromosome IX. One segregant that obtained all the markers from its parents (117 α) was again crossed with strain 10α, which contained 6 additional markers on chromosome V. The segregants of this latter cross were analysed by PCR for the presence of the parental markers.
The result of this analysis is shown in Fig. 10,B. Twenty segregants were analysed for the presence of the markers. This was done in a two-step approach in which all strains were first analysed for the presence of four markers, and only those that contained all of these markers were checked again for the presence of the remaining markers (note that strain 4A in the table, which contained only three of these four markers, was included in the final analysis). Strain STWW110 (corresponding to 6A in the table) was identified which had introgressed all seventeen markers from its parents. The results also reveal that crossing-over occurs frequently between adjacent markers, which facilitates the crossing-in of markers in adjacent regions on a chromosome and will increase the resolution of the mapping technology later on.
It is understood that the artificial markers are preferably introduced into silent regions in the genome, i.e. preferably in the non-transcribed region in between two adjacent genes. In this way, the phenotype of the resulting marker strain will not be affected by the presence of artificial DNA sequences. This is shown for the strain STWW110, in which 17 different marker sequences have been inserted in inter-generic regions on different chromosomes. Figure 10,C shows the results of a sensitive growth rate analysis of this strain and of the wild type strain using a Bioscreen apparatus (Labsystems). This method measures the increase in turbidity of a stationary-phase culture when incubated at 30°C. The original cultures were diluted into YPD up to an OD₆₀₀ of 0,05. Two duplicates of two independent cultures were measured simultaneously. The results show that there is no difference in the growth rate between the wild type strain and the seventeen-marker strain.

### Example 2 Construction of an artificially marked strain of the yeast Saccharomyces cerevisiae with markers having a rare-cutting restriction site adjacent to the marker and construction of a genomic library covering the whole yeast genome with specific marker-containing fragments.

The construction of such a strain is performed in the same way as described in Example 1 except that the oligonucleotide used as marker contains eight additional nucleotides specifying an Fsel restriction site 5' upstream of the specific oligonucleotide sequence. This is shown in Fig. 6.
A strain called STWW125-129, in the S288C background, with consecutive artificial markers with an Fsel restriction site (no. 125 to 129) has been deposited with the Belgian Coordinated Collection of Microorganisms (BCCM) (Brussels, Belgium) on June 21, 2001 under the Accession Number IHEM 18728, as mentioned above.
Another strain, called STWW110, and containing 17 markers with a Fsel recognition site, has been deposited by K.U.Leuven Research and Development with the Belgian Coordinated Collection of Microorganisms (BCCM) (Brussels, Belgium) on June 16, 2002 under the Accession Number IHEM 19413, as mentioned above.
For instance, in the case of the yeast S. cerevisiae, an Fsel site is added to the markers (see fig 6A). This enzyme cuts only about ten times in the yeast genomic sequence. When the genomic DNA in such a strain is digested with *Fse*l, genomic fragments are obtained which extend from an *Fse*l site to an adjacent *Fse*l site (Fig. 6,B). E.g. if the markers are introduced with 20 kb intervals, the *Fse*l-digestion fragments will be approximately 20 kb in size. The digestion products are subsequently cloned in a vector (see Fig. 6,C). This vector may be a plasmid, but for the cloning of 20 kb fragments it is preferred to use bacteriophage λ which may accommodate larger fragments of foreign DNA (up to 25 kb). After propagation in *E. coli,* the resulting transformants are analysed for the presence of the markers by PCR. The transformants are then stored in an ordered way for example in microtiter plates, starting with marker 1 and ending with the last marker that was inserted (Fig. 6,D). In this way, the whole genome is represented in the collection of transformants and each transformant contains a 20 kb fragment. Because every fragment contains a specific artificial marker and the presence of these markers can be analysed by PCR, this constitutes the first genomic library for which clear proof can be provided that every part of the genome is represented, both during construction, propagation and use of the library. This unique feature constitutes an important additional element of this invention. After transformation of the genomic library into a yeast strain, the DNA of the transformants can be pooled and the presence of the markers determined with the micro-array to check whether the whole genome is present in the collection of transformants. Once a particular phenotype is traced back to a mutation in a specific region of the genome, the corresponding 20 kb fragment containing the wild type gene can easily be taken from the sorted genomic library and transformed into the mutant strain for confirmation of the identification of the mapped genomic region by complementation.

### Example 3 Demonstration of linkage between a series of artificial markers and a mutation located close to the first marker of the series.

A strain in the S288C background with a *his3* mutation was used and 6 artificial markers were inserted adjacent to the *his3* locus with the procedure described in Example 1. The first marker was inserted at a distance of 20 kb from the *his3* locus. The other markers were inserted at 20 kb intervals from the previous marker.
The markers consisted of the following sequences: This artificially marked *his3* strain was crossed with a wild type strain in the S288C background, which has the wild type *HIS3* gene. The diploid strain was sporulated on sporulation medium and several asci were dissected with a micro-manipulator after zymolyase treatment of the ascus wall, according to standard procedures (Sherman et al. 1986, Methods in yeast genetics laboratory manual, Cold Spring Harbor Laboratory, Cold Spring Harbor, USA). The segregants were checked for histidine auxotrophy on medium lacking histidine. Twenty histidine minus (i.e. *his3*) segregants were then analysed for the presence of the 6 artificial markers using PCR. The two primers were the marker (see above) and an oligonucleotide with a sequence corresponding to a genomic sequence 200 to 800 kb downstream of the marker in the genome (checking primer). The sequences of the checking primers were as follows: The results were as follows:

| | No. of strains |
|---|---|
| Marker 1 | 20 |
| Marker 2 | 17 |
| Marker 3 | 7 |
| Marker 4 | 3 |
| Marker 5 | 7 |
| Marker 6 | 4 |

These results show that the closest marker, no.1, displays the strongest linkage to the *his3* mutation (all 20 segregants that have the mutation also contain the closest marker), also marker no.2 displays strong linkage with the *his3* mutation. Afterwards the linkage is lost. If there is no linkage between the *his3* mutation and a marker, statistically 50% of the *his3* strains should have the marker, the other 50% should lack it. The deviation from 50% in the current results is due to the low number of segregants investigated.
Because the first marker was introduced at a distance of 20 kb from the *his3* mutation, our results now confirm that strong linkage exists over a distance of 20 kb. Therefore, we can predict that in the application of the AMTEM™ technology, any mutation located in the unmarked strain in a position corresponding to the same position in the marked strain in between two markers spaced 20 kb apart, will make the markers disappear completely or nearly completely in the pooled DNA of the segregants with the mutation. As a result the position of the mutation will be indicated by a steep drop in signal intensity for at least the two markers located adjacent to the mutation.
In another experiment, a strain was used that carried 6 artificial markers and a mutation in the URA3 gene (*ura3*) which is located in between the last and the second last marker. This strain corresponds to strain 9a in Figure 9,A and it contains markers with the following sequences: The *ura3* mutation was located in between marker 153 and 154, at a distance of 13,7 kb from marker 153 and 5,5 kb from marker 154. This strain was crossed with a wild type strain containing the wild type URA3 gene and the segregants were analysed for growth on a medium lacking uracil. Twenty uracil minus (*ura3*) segregants were then analysed for the presence of the six markers by PCR, using the marker primers in combination with the following checking primers:

The results were as follows:

| | No. of *ura3* (-) strains |
|---|---|
| Marker 149 | 11 |
| Marker 150 | 15 |
| Marker 151 | 18 |
| Marker 152 | 19 |
| Marker 153 | 19 |

The results again show that the markers adjacent to the mutation (marker 153 and 154) are strongly linked to the mutation. Also the second closest marker (152) is strongly linked with the mutation in this example. The results also show that recombination between a mutation and the closest marker is possible but occurs at a low frequency. In this example, one crossing-over was observed between marker 153 and *ura3* and another between marker 154 and *ura3*. If the DNA of all the segregants that have gained the wild type URA3 gene through recombination are pooled, the analysis of the closest markers will reveal only a slight signal or no signal at all. The total number of crossing-overs within the region spanned by the six markers varied. Ten of the twenty segregants contained all six markers, presumably indicating that no crossing-over occurred within this region. In eight of the segregants crossing-over occurred once within the region spanned by the markers, and in one of the segregants even two crossing-overs occurred in this region. In addition to the strong linkage of a mutation with an adjacent marker located at a distance of less than 20 kb, the results demonstrate that crossing-overs within larger regions of the chromosomes (> 20 kb) occur with a sufficient frequency in order to be able to map the mutation of interest to a short genomic sequence. The latter region will generally correspond to the sequence in between two adjacent markers, but it may include a larger region as was the case in this example. If the marker analysis using pooled DNA from the segregants does not allow to locate the mutation in between two adjacent markers, more segregants should be included in the analysis or the approximate position of the mutation can be estimated based on the increase in marker intensity on both sides of the mutation (the mutation being generally located in the middle of this region). Alternatively, the segregants should be analysed individually for the presence of the markers, which will indicate in most cases the precise location of the mutation.
These results demonstrate for the first time that entirely artificial markers can be used for linkage analysis and they demonstrate for the first time the usefulness of a strain with a track of artificial markers for linkage analysis.

### Example 4 Demonstration of exclusion of a marker located adjacent to the his3 mutation and non-exclusion of a marker located adjacent to the unlinked ura3 mutation after crossing with an artificially marked strain.

A strain of the S288C background with a *his3* mutation and a *ura3* mutation was used. The *HIS3* and *URA3* genes are unlinked, *HIS3* is located on chromosome XV and *URA3* is located on chromosome V. Six artificial markers were inserted adjacent to the *his3* locus and four artificial markers were inserted adjacent to the *ura3* locus with the procedure described in Example 1 (see Fig. 12,A).

The first marker was inserted at a distance of 20 kb from the *his3* or *ura3* locus.
The other markers were inserted at 20 kb intervals from the previous marker. This strain is named STWW1 and has been deposited by Dr. Johan Thevelein, Dr. P. Ma and Dr. Patrick van Dijck (all from the Laboratory of Molecular Cell Biology, K.U.Leuven, Institute of Botany and Microbiology, Kasteelpark Arenberg 31, B-3001 Heverlee Belgium) with the Belgian Coordinated Collection of Microorganisms (BCCM) Scientific institute of Public Health- Louis Pasteur-Mycology IHEM (J. Wytsmanstraat 14, B 1050 Brussels, Belgium) on June 21, 2001 under the Accession Number IHEM 18730.
The markers adjacent to the *his3* mutation have been described above (EX. 3). The markers adjacent to the *ura3* mutation and the corresponding checking primers are as follows: This artificially marked *his3 ura3* strain was crossed with a wild type strain in the S288C background, which has the wild type *HIS3* and *URA3* genes. The diploid strain was sporulated on sporulation medium and several asci were dissected with a micro-manipulator after zymolyase treatment of the ascus wall, according to standard procedures (Sherman et al. 1986, Methods in yeast genetics laboratory manual, Cold Spring Harbor Laboratory, Cold Spring Harbor, USA). The segregants were checked for histidine and uracil auxotrophy on medium lacking histidine or uracil. Ten histidine plus (i.e. *HIS3*) segregants were then investigated for the presence of the 6 artificial markers adjacent to the *HIS3* locus and the 4 artificial markers adjacent to the *URA3* locus. The DNA of the segregants was extracted and pooled. The presence of the markers was detected using multiplex PCR amplification with either 100 ng or 10 ng of pooled DNA as template, single base extension using the four radioactive ³³P-dideoxynucleotides (see scheme in Fig. 11,A) and hybridisation onto a nylon membrane containing the complement of the markers. The results are shown in Fig. 12,8. They demonstrate that the marker closest to the *his3* mutation, marker no. 1, is totally excluded and that the next five markers,as well as the four markers adjacent to the *ura3* mutation can all be detected clearly. When 10 ng of genomic DNA is used as template for the multiplex PCR reaction (right panel), a gradient can be observed in the intensity of the signals starting from the *HIS3* locus. This gradient reflects the drop in linkage between the marker and the *his3* mutation.
In this experiment the markers were inserted next to the *his3* mutation and this strain was crossed with a wild type strain. This technology is referred to here as "Reverse AMTEM™". It is clear that insertion of the markers next to the *HIS3* wild type gene, crossing with a *his3* mutant and pooling of the DNA from the *his3* mutants would have excluded in exactly the same way the first marker from the pool of the DNA (this is the basic AMTEM™ technology). Hence, essentially the same result would have been obtained: the first marker indicates the position of the gene responsible for the phenotype of the segregants of which the DNA is pooled.
In a subsequent experiment the detection of the markers was performed by hybridisation to the complement of the markers spotted on a glass micro-array. In this case both the DNA from the *HIS3* segregants and the DNA from the *URA3* segregants was pooled and the presence of the markers detected. The results are shown in Fig. 12,C. They indicate that also in this case the marker closest to the *HIS3* locus is excluded when the DNA of the *HIS3* segregants is pooled and that the marker closest to the *URA3* locus is excluded when the DNA of the *URA3* segregants is pooled.
These results demonstrate for the first time that an artificial marker located close to the position of a mutation (defined as any change in the DNA sequence of the genome) is excluded and that artificial markers located farther away or on another chromosome are not excluded when the DNA of the mutant segregants from a cross between the mutant and the artificial marked strain is pooled. They demonstrate the usefulness of artificial markers for genetic mapping in isogenic strains and thus provide the proof of principle of the AMTEM™ technology.

### Example 5 Demonstration of exclusion of markers located close to two mutations with an unlinked position in the genome after crossing with an artificially marked strain.

The same strain as described in example 4 was used. This artificially marked *his3 ura3* strain was crossed with a wild type strain in the S288C background, which has the wild type *HIS3* and *URA3* genes. The diploid strain was sporulated on sporulation medium and several asci were dissected with a micro-manipulator after zymolyase treatment of the ascus wall, according to standard procedures (Sherman et al. 1986, Methods in yeast genetics laboratory manual, Cold Spring Harbor Laboratory, Cold Spring Harbor, USA). The segregants were checked for histidine and uracil auxotrophy on medium lacking histidine or uracil. Sixteen histidine plus (i.e. *HIS3*) and uracil plus (i.e. *URA3)* segregants were then investigated for the presence of the 6 artificial markers adjacent to the *HIS3* locus and the 4 artificial markers adjacent to the *URA3* locus. The DNA of the sixteen segregants was extracted and pooled. The presence of the markers was detected after multiplex PCR amplification, single base extension with a fluorescent dideoxynucleotide (labelled with Cy5), hybridisation to the complement of the markers spotted in micro-array format on a glass slide and detection by fluorescence scanning technology. The results are shown in Fig. 12,D.
These results demonstrate for the first time that entirely artificial markers can be used for the simultaneous mapping of multiple mutations required for a polygenic phenotype with a single cross of isogenic strains and they demonstrate for the first time the usefulness of a strain with a track of artificial markers for the simultaneous mapping of multiple mutations required for a polygenic phenotype with a single cross of isogenic strains.

### Example 6 Construction of strains containing multiple gene deletions in the genome.

The following haploid strains of the publicly available deletion strain collection have been used (Winzeler E.A. 1999, Science 285, 901-906) (The strains are named according to the open reading frame that is deleted.) YLR102C, YLL060C, YLL017W, YLR023C, YAR042W, YCL016C, YML058C-A, YOL047C, YLR205C, YOR382W, YLR224W, YOL008W
These strains were crossed two by two to generate six strains with two deletions, e.g. strain CAT1117 with deletions in YLR102C and YLL060C, strain CAT1118 with deletions in YLL017W and YLR023C, strain CAT1119 with deletions in YAR042W and YCL016C.
The double deletion strains were crossed to generate strains with four deletions; e.g. strain CAT1120 contains deletions in YLR102C, YLL060C, YLL017W, YLR023C.
In the next step sextuple deletion strains were constructed, e.g. the quadruple deletion strain CAT1120 was crossed with the double deletion strain CAT1119 to generate the strain CAT1121 which contains the six deletions present in its two parents.

In the next step ninefold deletion strains were constructed, e.g. the sextuple deletion strain CAT1121 was crossed with the sextuple deletion strain EVY5123 which has deletions in YML058C-A, YOL047C, YLR205C, YOR382W, YLR224W, YOL008W to generate the ninefold deletion strain EVYCAT1122 which has deletions in YLR102C, YLL060C, YLL017W, YLR023C, YAR042W, YCL016C, YLR205C, YLR224W, YOL008W.
In the next step a twelvefold deletion strain was constructed by crossing strain EVYCAT1122 with strain EVYCAT1123, which has deletions in YLR102C, YLL060C, YLR023C, YML058C-A, YOL047C, YLR205C, YOR382W, YLR224W, YOL008W, to generate strain EVYCAT1124-MATa which has deletions in the twelve open reading frames: YLR102C, YLL060C, YLL017W, YLR023C, YAR042W, YCL016C, YML058C-A, YOL047C, YLR205C, YOR382W, YLR224W, YOL008W.
The presence of the deletions was checked by PCR using as primers the specific oligonucleotide tags inserted with each deletion in the deletion strain collection ('downtag' and 'uptag') (Winzeler E.A. 1999, Science 285, 901-906). Detection by PCR of the twelve deletions in the strain EVYCAT1124-MATa is shown in Fig. 13. The strain EVYCAT1124-MATa has been deposited by Dr. Johan Thevelein, Dr. P. Ma and Dr. Patrick van Dijck (all from the Laboratory of Molecular Cell Biology, K.U.Leuven, Institute of Botany and Microbiology, Kasteelpark Arenberg 31, B-3001 Heverlee, Belgium) with the Belgian Coordinated Collection of Microorganisms (BCCM) Scientific Institute of Public Health- Louis Pasteur-Mycology IHEM (J. Wytsmanstraat 14, B 1050 Brussels, Belgium) on June 21, 2001 under the Accession Number IHEM 18729.
Figure 13 shows the presence of the mutations (deletions) in the strain (EVYCAT1124-MATa) is analysed by multiplex PCR, employing primer pairs for six ORF mutations in each reaction.
left lane: YLR102C, YLL060C, YLL017W, YLR023C, YAR042W, YCL016C. right lane: YML058C-A, YOL047C, YLR205C, YOR382W, YLR224W, YOL008W.

### Example 7 Generation of C. elegans strains for AMTEM™ gene mapping technology.

The AMTEM™ technology is applicable as well to other organisms apart from yeast. The availability of a sequenced genome greatly simplifies the engineering and evaluation of the introduction of markers. With the ever-increasing amount of genome sequence projects, the number of organisms that can be used for the AMTEM™ technology will increase as well.
In *C. elegans*, the oocytes of individual worms are injected with a mixture of specific oligonucleotides, ranging from a limited number up to the entire collection of 600 nucleotides that are being used in yeast. The DNA of the progeny is tested for the presence of the oligonucleotides with the use of the same technology as is being used for yeast. The organisms with the highest number of markers are selected. The DNA of the organism is fragmented and ligated into a vector. Sequences adjacent to a marker are amplified with the use of a primer in the marker and a primer in the vector, after which the sequence are determined. The markers that occur in neutral regions within genes are outcrossed. By subsequent crosses a worm is obtained which contains all the markers in neutral regions. In this way a worm with a limited number of markers is obtained. Further rounds of transformations and crosses are performed to obtain worms with a sufficient amount of markers to cover the entire genome and to perform the AMTEM™ and related technologies in *C. elegans*.
In an alternative approach a roundworm strain (RW7000) which has a high number of TC1 transposon insertions which are absent from the normal laboratory N2 strain is used. These transposons are active but can be stabilised by crossing in an additional mutation. The flanking sequence of a tranposon and its adjacent genomic sequence can be used as a specific marker. In a first attempt, the sequences of a limited number of these markers are applied on a micro-array. Probing the micro-array with genomic DNA of the RW7000 strain and genomic DNA of a control strain without transposons indicates the specificity of the markers. In a further stage more transposon insertions are sequenced and evaluated for their use as markers in an AMTEM™ mapping strategy. Transposon insertion has been shown to occur with a higher frequency in mutants that are defective in RNA silencing (Ketting R.F. et al., 1999, *mut-7* of *C. elegans*, required for transposon silencing and RNA interference, is a homolog of Werner syndrome helicase and RNase D, Cell 99, 133-141). This may be one of the possible approaches to develop strains in which the whole genome is covered with markers.

### Example 8 Generation of mouse strains for the AMTEM™ gene mapping technology.

The application of AMTEM™ technology for mouse will be facilitated when the genome sequence of mouse is completed. For practical reasons, the breeding of mice is limited and the introduction of markers is preferentially performed in embryonic stem (ES) cells.
Initially the same set of markers as being used in yeast are cloned in a vector next to a cassette with a neomycin resistance gene flanked by IoxP sites. Embryonic stem cells are transformed with a mixture of these constructs. Transformants are screened for the introduction of markers. Cells harbouring a large number of transformations are selected, after which the neomycine resistance gene is removed by the introduction of the Cre recombinase. This results in a scar of 34 basepairs adjacent to the specific oligonucleotide marker. The flanking sequences are determined in the same manner as was described for *C elegans*. By preference, cell lines are chosen where a minimal numbers of markers are introduced in, or nearby coding sequences.
The selected cell lines are used to generate mice. The embryonic stem cells of these mice are used again for a new round of introduction of markers. Mice with a sufficient number of markers are crossed to generate a new generation with a higher amount of markers. Finally a mouse is obtained with a sufficient amount of markers to cover the entire genome and to perform the AMTEM™ and its related technologies.
A more preferred strategy to generate mice marker strains is to transform ES cells derived from male cell lines, produce female mice by tetraploid embryo complementation (ES cell-tetraploid mice) from the cells that have undergone Y-chromosome loss, and use these to mate with ES cell-tetraploid males containing the same mutation(s), which results in homozygous mutant offspring after a single breeding cycle (Eggan et al., 2002, Male and female mice derived from the same embryonic cell clone by tetraploid embryo complementation, Nature Biotechnol. 20, 455-459). This strategy results in a substantial reduction of the time and expense required to produce mutant mouse strains carrying a large number of markers.

### Example 9 Generation of marker genotypes of plants for AMTEM™ gene mapping technology.

The genome sequence of model plant species such as *Arabidopsis thaliana* and rice has been determined recently and other genome sequences will become known in the near future. Several approaches can be used to generate plants that have markers along the genome that may be exploited through the AMTEM™ technology.
It is well known that several variants (ecotypes) of a same plant species have a considerable amount of variation in their genome. A number of these variations are deletions and insertions. By analysis of existing sequence databases we evaluate whether sufficient deletions or insertions occur that can be used as markers. In this case, a micro-array with oligonucleotides that cover these deletions and insertions is engineered. The value of this micro-array is evaluated with the DNA of the ecotypes used for the preparation of the array and with DNA of the progeny thereof.
In the eventual case that this variation is not sufficient, a similar approach as performed with *C. elegans* or mouse is performed. *Arabidopsis* plants are transformed with a mixture of a large number of oligonucleotides engineered into suitable transformation vectors. Plants with a high number of integrated oligonucleotides are selected with the microarray technology. The DNA sequences adjacent to the inserted oligonucleotides are determined at both sides. The markers that occur in neutral regions of the genome are outcrossed, and with new crosses a plant is generated that contains a maximal amount of markers in neutral regions. Also here additional round of transformations and crosses can be done to obtain a plant with the desired number and spacing of markers.
Particularly in *Arabidopsis,* large collections of mutant genotypes have been made through mutagenesis or random T-DNA or transposon tagging. In most cases, these plants have been generated in order to disrupt gene expression and obtain a selectable phenotype. However, many of these lines will also have insertions into non-coding regions and these may be employed as markers for mapping. Existing or generated collections of these mutants are therefore screened for the presence of genetic modifications in preferentially non-coding regions or new mutant genotypes are produced through transformation and/or mutagenesis. The screening is done by differential micro-array hybridisation to expressed genes (cDNAs) and to total DNA, and selection of those genotypes that only or preferentially hybridise to the latter micro-array. Alternatively, a micro-array is constructed that contains only or preferentially non-coding DNA, which may be obtained by the enrichment of random DNA sequences with non-coding DNA through subtraction with cDNA from various plant tissues. Such a non-coding DNA micro-array is then used for the detection of foreign DNA insertions and subsequent sequence analysis will reveal the position of the inserted DNA in the genome. Additional rounds of transformation and crossing the selected plants will then result in a plant with a large number of evenly spaced markers in the genome.
Alternatively, homologous recombination is used to insert marker sequences in the desired location in the genome. Gene targeting through homologous recombination is feasible but at present not yet very efficient in most plant species (Kempin et al., 1997, Targeted disruption in *Arabidopsis,* Nature 389, 802-803). Considerable efforts are being done to enhance the frequency of homologous recombination (Hanin et al., 2001, Gene targeting in *Arabidopsis,* Plant J. 28, 671-677). In combination with the high efficiency of transformation in *Arabidopsis* and an efficient screening system based on PCR and/or micro-array detection, the generation of plants with a large number of marker sequences through homologous recombination becomes feasible.
Homologous recombination at an efficiency comparable to that found for yeast is possible in the moss *Physcomitrella patens* (Schaeffer DG and Zryd JP, 1997, Efficient gene targeting in the moss *Physcomitrella patens,* Plant J. 11, 1195-1206). Once the whole genome sequence of this organism will become available, it is therefore possible to generate plants of this species that contain a large number of marker sequences and use these plants as a model marked genotype in genome mapping studies. As large parts of the genome will be conserved between moss and *Arabidopsis* or rice, it is also possible to select and sequence particular regions of the moss genome through homology with the known genome sequences and insert markers in those selected regions. In this case, it is not necessary to have the whole moss genome sequence determined.
It is known that the integration of foreign DNA through homologous recombination is linked with the mismatch repair system that is used by cells to repair DNA double-stranded breaks. Understanding why homologous recombination is so efficient in this plant species, e.g. through targeting the genes of the mismatch repair system, could help to elucidate control of homologous recombination in other plants. It is understood that the strategy used to generate marker genotypes in plants is not essential to this invention and that any improvement in the techniques used to do so will have no effect on the validity of this patent.

### SEQUENCE LISTING

<110> K.U.Leuven Research & Development vzw
   Thevelein, Johan
   Van Dijck, Patrick
   Ma, Pingsheng
   Dumortier, Françoise
   Broothaerts, Wim
<120> novel technology for genetic mapping
<130> K-2037 PCT
<150> UK 0115194.3
   <151> 2001-06-21
<160> 74
<170> PatentIn version 3.1
<210> 1
   <211> 19
   <212> DNA
   <213> synthetic primer
<400> 1
   cgaattccag ctgaccacc
   19
<210> 2
   <211> 20
   <212> DNA
   <213> synthetic primer
<400> 2
   gatccccggg aattgccatg
   20
<210> 3
   <211> 19
   <212> DNA
   <213> synthetic primer
<400> 3
   aatgcacgtc aacagcacg
   19
<210> 4
   <211> 39
   <212> DNA
   <213> synthetic primer
<400> 4
   cgaattccag ctgaccaccg ctagagcaga agaacaggg
   39
<210> 5
   <211> 48
   <212> DNA
   <213> synthetic primer
<400> 5
   gcgtgctgtt gacgtgcatt ggccggccga gtcatggcta ctatatgg 48
<210> 6
   <211> 49
   <212> DNA
   <213> synthetic primer
<400> 6
   ggccggccaa tgcacgtcaa cagcacgccg atggacttaa agaaccagg 49
<210> 7
   <211> 40
   <212> DNA
   <213> synthetic primer
<400> 7
   gatccccggg aattgccatg ttgtcctttc catgatgccg 40
<210> 8
   <211> 20
   <212> DNA
   <213> synthetic primer
<400> 8
   gcagcccaga agggaaatgg 20
<210> 9
   <211> 19
   <212> DNA
   <213> synthetic primer
<400> 9
   ctgcaaacaa atgaggcgg 19
<210> 10
   <211> 39
   <212> DNA
   <213> synthetic primer
<400> 10
   cgaattccag ctgaccacca tggcctacca cctggaagg 39
<210> 11
   <211> 48
   <212> DNA
   <213> synthetic primer
<400> 11
   gccgcctcat ttgtttgcag ggccggccga tggattctcg ttcgctag
   48
<210> 12
   <211> 49
   <212> DNA
   <213> synthetic primer
<400> 12
   ggccggccct gcaaacaaat gaggcggcat aacttcgtca ttcagtgcg
   49
<210> 13
   <211> 40
   <212> DNA
   <213> synthetic primer
<400> 13
   gatccccggg aattgccatg agaaagagga gcaggcacag
   40
<210> 14
   <211> 20
   <212> DNA
   <213> synthetic primer
<400> 14
   ttgagatact ctgcgttggg
   20
<210> 15
   <211> 19
   <212> DNA
   <213> synthetic primer
<400> 15
   aggcgtccga taactagag
   19
<210> 16
   <211> 40
   <212> DNA
   <213> synthetic primer
<400> 16
   cgaattccag ctgaccaccg aaagtatatg gtgagtcctc 40
<210> 17
   <211> 48
   <212> DNA
   <213> synthetic primer
<400> 17
   gctctagtta tcggacgcct ggccggccca tatacgagtg gtccgacg 48
<210> 18
   <211> 50
   <212> DNA
   <213> synthetic primer
<400> 18
   ggccggccag gcgtccgata actagagcca ttttcttttg gatcacaccc 50
<210> 19
   <211> 40
   <212> DNA
   <213> synthetic primer
<400> 19
   gatccccggg aattgccatg ttaccaccaa tgcctacgtc 40
<210> 20
   <211> 20
   <212> DNA
   <213> synthetic primer
<400> 20
   gagtcttctg taatggctgc 20
<210> 21
   <211> 19
   <212> DNA
   <213> synthetic primer
<400> 21
   gctcgtccct taattagcg
   19
<210> 22
   <211> 39
   <212> DNA
   <213> synthetic primer
<400> 22
   cgaattccag ctgaccaccg gttttcatta ccctatcac
   39
<210> 23
   <211> 48
   <212> DNA
   <213> synthetic primer
<400> 23
   ccgctaatta agggacgagc ggccggccca tctttttgtt aggggcca
   48
<210> 24
   <211> 48
   <212> DNA
   <213> synthetic primer
<400> 24
   ggccggccgc tcgtccctta attagcgggc aaggattgaa ataatccg
   48
<210> 25
   <211> 40
   <212> DNA
   <213> synthetic primer
<400> 25
   gatccccggg aattgccatg aaaacccacg agccaacaac
   40
<210> 26
   <211> 20
   <212> DNA
   <213> synthetic primer
<400> 26
   tagactgcta ggccaatacc
   20
<210> 27
   <211> 20
   <212> DNA
   <213> synthetic primer
<400> 27
   gcaagactta agtcaccggc
   20
<210> 28
   <211> 39
   <212> DNA
   <213> synthetic primer
<400> 28
   cgaattccag ctgaccacct tagccattga tgcgtcacc
   39
<210> 29
   <211> 50
   <212> DNA
   <213> synthetic primer
<400> 29
   agccggtgac ttaagtcttg cggccggccc caggcaaata aaagggagag
   50
<210> 30
   <211> 49
   <212> DNA
   <213> synthetic primer
<400> 30
   ggccggccgc aagacttaag tcaccggctc tttggtgtct catagcttc
   49
<210> 31
   <211> 40
   <212> DNA
   <213> synthetic primer
<400> 31
   gatccccggg aattgccatg ctaacagaac gcataagtcc
   40
<210> 32
   <211> 20
   <212> DNA
   <213> synthetic primer
<400> 32
   actatgatgt tggtcacagc
   20
<210> 33
   <211> 34
   <212> DNA
   <213> synthetic primer
<400> 33
   catggcaatt cccggggatc gtgattctgg gtag
   34
<210> 34
   <211> 21
   <212> DNA
   <213> synthetic primer
<400> 34
   ttgacgttcg ttcgactgat g
   21
<210> 35
   <211> 22
   <212> DNA
   <213> synthetic primer
<400> 35
   gagcaatgaa cccaataacg aa
   22
<210> 36
   <211> 37
   <212> DNA
   <213> synthetic primer
<400> 36
   ggtggtcagc tggaattcga tgatgtagtt tctggtt
   37
<210> 37
   <211> 20
   <212> DNA
   <213> synthetic primer
<400> 37
   ggctaatagc ctattgcggc
   20
<210> 38
   <211> 39
   <212> DNA
   <213> synthetic primer
<400> 38
   cgaattccag ctgaccacct cttggagaag aagagacgg
   39
<210> 39
   <211> 48
   <212> DNA
   <213> synthetic primer
<400> 39
   agccgcaata ggctattagc cggccggatg tcattgactt gacttgga
   48
<210> 40
   <211> 54
   <212> DNA
   <213> synthetic primer
<400> 40
   ggccggccgg ctaatagcct attgcggctg agagtgcata tatacattgt tgga
   54
<210> 41
   <211> 40
   <212> DNA
   <213> synthetic primer
<400> 41
   gatccccggg aattgccatg tcaactaatc gatggtccag
   40
<210> 42
   <211> 20
   <212> DNA
   <213> synthetic primer
<400> 42
   cactttgggt ctgtatagcg
   20
<210> 43
   <211> 20
   <212> DNA
   <213> synthetic primer
<400> 43
   ggatgcacag cagacattcc
   20
<210> 44
   <211> 21
   <212> DNA
   <213> synthetic primer
<400> 44
   attctgatgc ttacgtcggt g
   21
<210> 45
   <211> 20
   <212> DNA
   <213> synthetic primer
<400> 45
   atactgacag catgcatggc
   20
<210> 46
   <211> 20
   <212> DNA
   <213> synthetic primer
<400> 46
   agtgttcaag caggactgtg
   20
<210> 47
   <211> 20
   <212> DNA
   <213> synthetic primer
<400> 47
   tacagatcag cagacatggc
   20
<210> 48
   <211> 20
   <212> DNA
   <213> synthetic primer
<400> 48
   atttgggtaa gcgtatcgcc
   20
<210> 49
   <211> 20
   <212> DNA
   <213> synthetic primer
<400> 49
   cggcatacta cacagagtcc
   20
<210> 50
   <211> 20
   <212> DNA
   <213> synthetic primer
<400> 50
   acttcataga ggtgcacccg
   20
<210> 51
   <211> 20
   <212> DNA
   <213> synthetic primer
<400> 51
   cgatgatcca tacgcagtcc
   20
<210> 52
   <211> 20
   <212> DNA
   <213> synthetic primer
<400> 52
   gtttgcatta gggagaccgg
   20
<210> 53
   <211> 20
   <212> DNA
   <213> synthetic primer
<400> 53
   gcgaaattgc gtcaagctcc
   20
<210> 54
   <211> 20
   <212> DNA
   <213> synthetic primer
<400> 54
   gagtaccccc aacaacgatg
   20
<210> 55
   <211> 20
   <212> DNA
   <213> synthetic primer
<400> 55
   gctataggtc aaccagccac
   20
<210> 56
   <211> 20
   <212> DNA
   <213> synthetic primer
<400> 56
   tagtcaaagg ttggaaggcg
   20
<210> 57
   <211> 20
   <212> DNA
   <213> synthetic primer
<400> 57
   cgcagttcat gactaggcac
   20
<210> 58
   <211> 20
   <212> DNA
   <213> synthetic primer
<400> 58
   tgtccatgtt gctagaagcc
   20
<210> 59
   <211> 20
   <212> DNA
   <213> synthetic primer
<400> 59
   gagtactcag aagctcagac
   20
<210> 60
   <211> 20
   <212> DNA
   <213> synthetic primer
<400> 60
   aagcctaagt ggagctgatg
   20
<210> 61
   <211> 20
   <212> DNA
   <213> synthetic primer
<400> 61
   gcactatgag taggcatagc
   20
<210> 62
   <211> 20
   <212> DNA
   <213> synthetic primer
<400> 62
   aagcctacgc atgatgtagg
   20
<210> 63
   <211> 20
   <212> DNA
   <213> synthetic primer
<400> 63
   aactggacca actaagccgc
   20
<210> 64
   <211> 20
   <212> DNA
   <213> synthetic primer
<400> 64
   aaaacacgtt caaccggggc
   20
<210> 65
   <211> 20
   <212> DNA
   <213> synthetic primer
<400> 65
   accttcataa atccgggccg
   20
<210> 66
   <211> 20
   <212> DNA
   <213> synthetic primer
<400> 66
   catttccaac agccggaacg
   20
<210> 67
   <211> 20
   <212> DNA
   <213> synthetic primer
<400> 67
   tgtgtcggta actacgcagc
   20
<210> 68
   <211> 20
   <212> DNA
   <213> synthetic primer
<400> 68
   ttacctccac taagcgtgcc
   20
<210> 69
   <211> 20
   <212> DNA
   <213> synthetic primer
<400> 69
   aaagaaaaat gggccggcag 20
<210> 70
   <211> 20
   <212> DNA
   <213> synthetic primer
<400> 70
   ctacaggagc atggaaatgg 20
<210> 71
   <211> 20
   <212> DNA
   <213> synthetic primer
<400> 71
   aagcctaagt ggagctgatg 20
<210> 72
   <211> 20
   <212> DNA

## Claims

1. A strain of a non human eukaryotic organism whose genome has been modified by man made intervention, to have a plurality of one or more modifications distributed substantially equally throughout a part, several parts, or the entire length of one or more or all of the chromosomes of the genome, the modifications resulting in a plurality of different marker sequences each being unique with respect to the unmodified genome.

2. A strain of a non human eukaryotic organism according to claim 1,
wherein the modifications are outside any coding region of a gene and outside any regulatory parts of a gene.

3. A strain of a non human eukaryotic organism according to claim 1 or 2,
wherein the modifications occur at a rate of at least 1 modification / 100 genes.

4. The strain according to any one of claims 1 to 3, wherein the modifications are site specific or site directed.

5. The strain according to any one of claims 1 to 4, the modifications not affecting the phenotype in comparison with the unmodified organism.

6. The strain according to any one of claims 1 to 5, which is viable and able to reproduce sexually.

7. The strain according to any one of claims 1 to 6, in which the modification is
a) a deletion or an insertion or a substitution, being selected from one or more of the following:
- the deletion or insertion or substitution as such;
- the deletion or insertion or substitution flanked by one or more restrictions sites;
- the deletion or insertion or substitution by one or more nucleotide tags;
- the deletion or insertion or substitution flanked with inverted repeats
- the deletion or insertion or substitution flanked with inverted repeats of a transposon;
- the deletion or insertion or substitution flanked with the long terminal repeats of a retrovirus
- a sequence flanked with recognition sites for a recombinase adjacent to the deletion or insertion or substitution;
- said deletion or insertion or a substitution flanked at one or both sides with genomic sequence, the said genomic sequence containing one or more insertions or deletions or substitutions; or
b) a naturally occurring mobile genetic element or the footprint after excision of said mobile genetic element.

8. The strain according to any one of claims 1 to 7, wherein the modifications are introduced by a method selected from the group consisting of homologous recombination, transposition, viral infection, random integration with subsequent selection and *Agrobacterium* mediated DNA integration or a process analogous herewith.

9. The strain according to any one of claims 1 to 8, wherein said organism is selected from the group consisting of fungi, non vascular plants, vascular plants, arthropods, nematodes, vertebrates, mammals, rodents.

10. The strain according to any one of claims 1 to 9, said organism being selected from *Saccharomyces cerevisiae, Schizosaccharomyces pombe Asspegillus nidulans, Neurospora sp., Caenorhabditis elegans,* *Physcomitrella sp., Arabidopsis thaliana, Oryza sativa, Drosophila melanogaster, Brachydanio rerio, Mus musculus.*

11. A method for gene mapping comprising the steps of:
a. crossing a strain of a first non-human eukaryotic organism of which the genome has been modified, to have a plurality of one or more modifications distributed substantially equally throughout a part, several parts or the entire length of one or more or all of the chromosomes of the genome, the modifications resulting in a plurality of different marker sequences each being unique with respect to the unmodified genome, with a second strain of said non-human eukaryotic organism with a phenotype of interest differing from said first strain of said non-human eukaryotic organism;
b. selecting segregants of the crossing in (a) with the said phenotype of interest;
c. isolating DNA from segregants selected under (b);
d. optionally pooling the isolated DNA;
e. detecting the occurrence of at least one marker sequence in said DNA; and
f. mapping one or more genes responsible for said phenotype of interest based on the absence of said at least one marker sequence.

12. The method of claim 11, in which the detection of marker sequences is performed by hybridisation or by polymerase chain reaction.

13. A method for gene mapping comprising the steps of:
a) generating mutations in a first non-human eukaryotic organism until a phenotype of interest is obtained,
wherein said first non-human eukaryotic organism is a strain of an organism whose genome has been modified by man made intervention, to have a plurality of one or more modifications distributed substantially equally throughout a part, several parts, or the entire length of one or more or all of the chromosomes of the genome, the modifications resulting in a plurality of different marker sequences each being unique with respect to the unmodified genome.
b) crossing the organism of (a) with the phenotype of interest with a second wild type strain,
c) selecting segregants of the crossing in (b) which are wild type for the phenotype of interest
d) isolating DNA from the selected segregants of step (c) and pooling the DNA.
e) detecting the presence of a marker sequence present in the pooled DNA of step (d)
f) mapping the position of a mutation causing the phenotype of interest by the absence of markers, said markers being located closest to said mutation.

14. The method according to claim 13, wherein the ratio of generated mutations versus the number of non essential genes in said strain is at least 0,5 percent.

15. The method according to claim 13 or 14, wherein said eukaryotic organism is a fungus.

16. The method according to any one of claims 13 to 15, wherein said eukaryotic organism is *Saccharomyces cerevisiae*.

17. The method according to according to claim 11 or 13, wherein said first non-eukaryotic organism is obtained by mutagenesis, inactivation or deletion of genes in a strain.

18. A set of oligonucleotides or their complements, or a number of these oligonucleotides or their complements for the production of a strain of an organism according to claim 1.

19. The set of oligonucleotides of claim 18, applied on a carrier or micro-array.

20. The method according to claim 11 or 13, which further comprises the steps of isolating and purifying said mapped gene(s) and, optionally, further comprising the step of introducing the mapped gene into a vector.

## Patentansprüche

1. Stamm eines nicht-menschlichen eukaryontischen Organismus, dessen Genom durch menschlichen Eingriff modifiziert wurde, so dass es eine Vielzahl einer oder mehrerer Modifikationen aufweist, die im Wesentlichen gleich in einem Teil, mehreren Teilen oder in der gesamten Länge einer oder mehrerer oder aller Chromosomen des Genoms verteilt sind, wobei die Modifikationen eine Vielzahl unterschiedlicher Marker-Sequenzen zur Folge haben, die bezüglich des unmodifizierten Genoms einmal vorkommen.

2. Stamm eines nicht-menschlichen eukaryontischen Organismus nach Anspruch 1, wobei die Modifikationen außerhalb der kodierenden Region eines Genes und außerhalb aller regulatorischen Teile eines Genes liegen.

3. Stamm eines nicht-menschlichen eukaryontischen Organismus nach Anspruch 1 oder 2, wobei die Modifikationen in einer Rate von zumindest 1 Modifikation/100 Genen auftreten.

4. Stamm nach einem der Ansprüche 1 bis 3, wobei die Modifikationen ortspezifisch oder ortsgerichtet sind.

5. Stamm nach einem der Ansprüche 1 bis 4, wobei die Modifikationen den Phänotyp im Vergleich mit dem unmodifizierten Organismus nicht beeinträchtigen.

6. Stamm nach einem der Ansprüche 1 bis 5, der lebensfähig ist und dazu in der Lage ist, sich sexuell zu reproduzieren.

7. Stamm nach einem der Ansprüche 1 bis 6, bei dem die Modifikation Folgendes ist:
a) eine Deletion oder eine Insertion oder eine Substitution, ausgewählt aus einem oder mehreren des nachfolgenden:
- der Deletion oder Insertion oder Substitution als solches;
- der Deletion oder Insertion oder Substitution, flankiert von ein oder mehreren Restriktionsorten;
- der Deletion oder Insertion oder Substitution durch ein oder mehrere Nukleotid-Markierungen;
- der Deletion oder Insertion oder Substitution, flankiert durch inverted repeats;
- der Deletion oder Insertion oder Substitution, flankiert durch inverted repeats eines Transposons;
- der Deletion oder Insertion oder Substitution, flankiert mit den langen terminalen Wiederholungen (LTR) eines Retrovirus;
- einer Sequenz, die durch Erkennungsorte für eine Rekombinase flankiert ist, die der Deletion oder Insertion oder Substitution benachbart sind;
- wobei die Deletion oder Insertion oder Substitution an einem oder beiden Seiten mit einer genomischen Sequenz flankiert ist, wobei die genomische Sequenz eine oder mehrere Insertionen oder Deletionen oder Substitutionen enthält; oder
b) ein natürlich vorkommendes mobiles genetisches Element oder der Footprint nach Exzision des mobilen genetischen Elementes.

8. Stamm nach einem der Ansprüche 1 bis 7, wobei die Modifikationen durch ein Verfahren eingebracht werden, ausgewählt aus der Gruppe, die aus homologer Rekombination, Transposition, Virusinfektion, Zufalls-bedingter Integration mit anschließender Selektion und Agrobacterium-vermittelter DNA-Integration oder einem hierzu analogen Verfahren besteht.

9. Stamm nach einem der Ansprüche 1 bis 8, wobei der Organismus aus der Gruppe ausgewählt ist, die aus Pilzen, gefäßfreien Pflanzen, Gefäßpflanzen, Arthropoden, Nematoden, Vertebraten, Säugetieren, Nagetieren besteht.

10. Stamm nach einem der Ansprüche 1 bis 9, wobei der Organismus aus *Saccharomyces cerevisiae, Schizosaccharomyces pombe, Asspegillus nidulans, Neurospora sp., Caenorhabditis elegans, Physcomitrella sp., Arabidopsis thaliana; Oryza sativa, Drosophila melanogaster, Brachydanio rerio, Mus musculus* ausgewählt ist.

11. Verfahren zur Genkartierung, das die folgenden Schritte umfasst:
a) Kreuzen eines Stammes eines ersten nicht-menschlichen eukaryomischen Organismus, dessen Genom modifiziert wurde, so dass es eine Vielzahl einer oder mehrerer Modifikationen im Wesentlichen gleichmäßig über einen Teil, mehrere Teile oder über die Gesamtlänge eines oder mehrerer oder aller Chromosomen des Genoms verteilt aufweist, wobei die Modifikation eine Vielzahl unterschiedlicher Marker-Sequenzen zur Folge haben, die jeweils im Hinblick auf das nicht modifizierte Genom einmal vorkommen, mit einem zweiten Stamm des nicht-menschlichen eukaryontischen Organismus mit einem Phänotyp von Interesse, der sich vom ersten Stamm des nicht-menschlichen eukaryontischen Organismus unterscheidet;
b) Auswählen von Abkömmlingen des Kreuzens in (a) mit dem zweiten Phänotyp von Interesse;
c) Isolieren einer DNA aus den unter (b) ausgewählten Abkömmlingen;
d) Wahlweises Poolen der isolierten DNA;
e) Nachweisen des Auftretens zumindest einer Marker-Sequenz in dieser DNA; und
f) Kartieren eines oder mehrerer Gene, die für diesen Phänotyp von Interesse verantwortlich sind, auf Grundlage des Fehlens der zumindest einen Marker-Sequenz.

12. Verfahren nach Anspruch 11, bei dem der Nachweis von Marker-Sequenzen durch Hybridisierung oder durch Polymerase-Kettenreaktion durchgeführt wird.

13. Verfahren zur Gen-Kartierung, das die folgenden Schritte umfasst:
a) Erzeugung von Mutationen in einem ersten nicht-humanen eukaryontischen Organismus bis ein Phänotyp von Interesse erzielt wird, wobei der erste nichtmenschliche eukaryontische Organismus ein Stamm eines Organismus ist, dessen Genom durch menschlichen Eingriff modifiziert wurde, so dass es eine Vielzahl einer oder mehrerer Modifikationen aufweist, die im Wesentlichen gleichmaßig in einem Teil, mehreren Teilen oder der gesamten Länge eines oder mehrerer oder aller Chromosomen des Genoms verteilt sind, wobei die Modifikation eine Vielzahl unterschiedlicher Marker-Sequenzen zur Folge haben, die jeweils bezüglich des unmodifizierten Genoms einmal vorkommen,
b) Kreuzen des Organismus aus (a) mit dem Phänotyp von Interesse mit einem zweiten Wild-Typ-Stamm,
c) Auswählen von Abkömmlingen von Kreuzung in (b), die für den Phänotyp von Interesse Wild-Typ sind,
d) Isolieren von DNA aus den ausgewählten Abkömmlingen aus Schritt (c) und Poolen der DNA,
e) Nachweisen des Vorhandenseins einer Marker-Sequenz, die in der gepoolten DNA aus Schritt (d) vorliegt, und
f) Kartieren der Position einer Mutation, die den Phänotyp von Interesse verursacht, durch die Abwesenheit von Markern, wobei die Marker sich am nächsten zur Mutation befinden.

14. Verfahren nach Anspruch 13, wobei das Verhältnis der erzeugten Mutationen gegen die Anzahl nicht essentieller Gene in diesem Stamm zumindest 0,5% beträgt.

15. Verfahren nach Anspruch 13 oder 14, wobei der eukaryontische Organismus ein Pilz ist.

16. Verfahren nach einem der Ansprüche 13 bis 15, wobei der eukaryontische Organismus *Saccharomyces cerevisae* ist.

17. Verfahren nach Anspruch 11 oder 13, wobei der erste nicht eukaryontische Organismus durch Mutagenese, Inaktivierung oder Deletion von Genen in einem Stamm gewonnen wird.

18. Satz Oligonukleotide oder deren Komplemente oder mehrere dieser Oligonukleotide oder ihrer Komplemente zur Produktion eines Stammes eines Organismus nach Anspruch 1.

19. Satz von Oligonukleotiden nach Anspruch 18, der auf einen Träger oder ein Mikro-Array aufgebracht ist.

20. Verfahren nach Anspruch 11 oder 13, das die Schritte des Isolierens und Aufreinigens der kartierten Gene und wahlweise weiterhin den Schritt umfasst, das kartierte Gen in einen Vektor einzubringen.

## Revendications

1. Une souche d'un organisme eucaryote non humain dont le génome a été modifié par intervention de la main de l'homme, pour avoir une pluralité d'une ou plusieurs modifications distribuées de manière substantiellement égale à travers une partie, plusieurs parties, ou toute la longueur d'un ou plusieurs ou de tous les chromosomes du génome, les modifications ayant pour résultat une pluralité de différentes séquences de marqueurs, chacune étant unique par rapport au génome non modifié.

2. Une souche d'un organisme eucaryote non humain selon la revendication 1, dans laquelle les modifications sont à l'extérieur d'une quelconque région de codage d'un gène et à l'extérieur de quelconques parties régulatrices d'un gène.

3. Une souche d'un organisme eucaryote non humain selon la revendication 1 ou 2, dans laquelle les modifications se produisent à un taux d'au moins une modification pour 100 gènes.

4. La souche selon l'une quelconque des revendications 1 à 3, dans laquelle les modifications sont spécifiques d'un site ou dirigées sur un site.

5. La souche selon l'une quelconque des revendications 1 à 4, les modifications n'affectant pas le phénotype par comparaison à l'organisme non modifié.

6. La souche selon l'une quelconque des revendications 1 à 5, qui est viable et apte à se reproduire sexuellement.

7. La souche selon l'une quelconque des revendications 1 à 6, dans laquelle la modification est
a) une suppression ou une insertion, étant sélectionnée à partir de l'une ou de plusieurs des suivantes :
- la suppression ou l'insertion ou la substitution en tant que telle ;
- la suppression ou l'insertion ou la substitution flanquée par un ou plusieurs sites de restriction ;
- la suppression ou l'insertion ou la substitution par un ou plusieurs tags de nucléotides ;
- la suppression ou l'insertion ou la substitution flanquée par des répétitions inversées ;
- la suppression ou l'insertion ou la substitution flanquée par des répétitions inversées d'un transposon ;
- la suppression ou l'insertion ou la substitution flanquée par les répétitions terminales longues d'un rétrovirus ;
- une séquence flanquée par des sites de reconnaissance pour une recombinase adjacente à la suppression ou l'insertion ou la substitution ;
- ladite suppression ou insertion ou substitution flanquée d'un côté ou des deux côtés par une séquence génomique, ladite séquence génomique contenant une ou plusieurs suppressions ou insertions ou substitutions ; ou
b) un élément génétique mobile d'occurrence naturelle ou l'empreinte après excision dudit élément génétique mobile.

8. La souche selon l'une quelconque des revendications 1 à 7, dans laquelle les modifications sont introduites par une méthode sélectionnée parmi le groupe constitué de la recombinaison homologue, la transposition, l'infection virale, l'intégration au hasard avec sélection subséquente et l'intégration d'ADN par l'intermédiaire *d'Agrobacterium* ou un procédé analogue à celui-ci.

9. La souche selon l'une quelconque des revendications 1 à 8, dans laquelle ledit organisme est sélectionné parmi le groupe constitué des champignons, des plantes non vasculaires, des plantes vasculaires, des arthropodes, des nématodes, des vertébrés, des mammifères, et des rodeurs.

10. La souche selon l'une quelconque des revendications 1 à 9, ledit organisme étant sélectionné à partir de *Saccharomyces cerevisiae, Schizosaccharomyces pombe, Asspegillus nidulans, Neurospora sp., Caenorhabditis elegans, Physcomitrella sp., Arabidopsis thaliana, Oryza sativa, Drosophila melanogaster, Brachydanio rerio, Mus musculus.*

11. Une méthode de cartographie génétique comprenant les étapes consistant à :
a. croiser une souche d'un premier organisme eucaryote non humain dont le génome a été modifié, pour avoir une pluralité d'une ou plusieurs modifications distribuées de manière substantiellement égale à travers une partie, plusieurs parties, ou toute la longueur d'un ou de plusieurs ou de tous les chromosomes du génome, les modifications résultant en une pluralité de différentes séquences de marqueurs, chacune étant uni-que par rapport au génome non modifié, avec une seconde souche dudit organisme eucaryote non humain ayant un phénotype d'intérêt différent de ladite première souche dudit organisme eucaryote non humain ;
b. sélectionner des ségrégants du croisement en (a) avec ledit phénotype d'intérêt ;
c. isoler l'ADN des ségrégants sélectionnés en (b) ;
d. optionnellement regrouper l'ADN isolé ;
e. détecter l'occurrence d'au moins une séquence de marqueur dans ledit ADN ; et
f. cartographier un ou plusieurs gènes responsables dudit phénotype d'intérêt sur la base de l'absence de ladite au moins une séquence de marqueur.

12. La méthode de la revendication 11, dans laquelle la détection des séquences de marqueurs est effectuée par hybridisation ou par réaction de polymérase en chaîne.

13. Une méthode de cartographie génétique comprenant les étapes consistant à :
a) générer des mutations dans un premier organisme eucaryote non humain jusqu'à ce qu'un phénotype d'intérêt soit obtenu,
dans lequel ledit premier organisme eucaryote non humain est une souche d'un organisme dont le génome a été modifié par intervention de la main de l'homme, pour avoir une pluralité d'une ou plusieurs modifications distribuées de manière substantiellement égale à travers une partie, plusieurs parties, ou toute la longueur d'un ou plusieurs ou de tous les chromosomes du génome, les modifications résultant en une plura-lité de différentes séquences de marqueurs, chacune étant unique par rapport au génome non modifié,
b) croiser l'organisme de (a) ayant le phénotype d'intérêt avec une seconde souche du type sauvage,
c) sélectionner des ségrégants du croisement en (b) qui sont du type sauvage pour le phénotype d'intérêt,
d) isoler l'ADN à partir des ségrégants sélectionnés dans l'étape (c) et regrouper l'ADN,
e) détecter la présence d'une séquence de marqueur présente dans l'ADN regroupé dans l'étape (d),
f) cartographier la position d'une mutation provoquant le phénotype d'intérêt par l'absence des marqueurs, lesdits marqueurs étant situés le plus près de ladite mutation.

14. La méthode selon la revendication 13, dans laquelle le rapport des mutations générées vis à vis du nombre de gènes non essentiels dans ladite souche est au moins 0,5 pourcent.

15. La méthode selon la revendication 13 ou 14, dans laquelle ledit organisme eucaryote est un champignon.

16. La méthode selon l'une quelconque des revendications 13 à 15, dans laquelle ledit organisme eucaryote est *Saccharomyces cerevisiae*.

17. La méthode selon la revendication 11 ou 13, dans laquelle ledit premier organisme non eucaryotique est obtenu par mutagénèse, inactivation ou suppression de gènes dans une souche.

18. Un ensemble d'oligonucléotides ou leurs compléments, ou un nombre de ces oligonucléotides ou leurs compléments, pour la production d'une souche d'un organisme selon la revendication 1.

19. L'ensemble d'oligonucléotides de la revendication 18, appliqué sur un support ou une micro-collection.

20. La méthode selon la revendication 11 ou 13, qui comprend en outre les étapes consistant à isoler et à purifier ledit (lesdits) gène(s) cartographié(s) et, optionnellement, comprenant en outre l'étape consistant à introduire le gène cartographié dans un vecteur.
